# EUROPEAN PATENT SPECIFICATION

(11) **EP 4 524 223 B1**
(45) Date of publication and mention of the grant of the patent: **03.06.2026**
(21) Application number: 23823791.1
(22) Date of filing: 07.06.2023
(51) Int. Cl.: C07C 69/76, C11B 9/00, C07C 47/228, C07C 45/62, C07B 61/00

(54) **ALDEHYDE COMPOSITION**
ALDEHYDZUSAMMENSETZUNG
COMPOSITION D'ALDÉHYDE

(30) Priority: 17.06.2022 JP 2022098365
(43) Date of publication of application: 19.03.2025
(73) Proprietor: MITSUBISHI GAS CHEMICAL COMPANY, INC., Chiyoda-ku Tokyo 100-8324 (JP)
(72) Inventor: HAYASHI, Masaki, Kurashiki-shi, Okayama 712-8525 (JP); MATSUDA, Daiki, Kurashiki-shi, Okayama 712-8525 (JP); NISHIUCHI, Junya, Kurashiki-shi, Okayama 712-8525 (JP)
(74) Representative: Müller-Boré & Partner Patentanwälte PartG mbB
(86) International application number: PCT/JP2023/021085
(87) International publication number: WO 2023/243499

(56) References cited:
- WO-A1-2021/075517
- FR-A- 1 460 826
- JP-A- 2016 523 828
- JP-A- 2016 530 227
- JP-A- 2018 502 057
- JP-A- 2018 523 641
- JP-A- 2018 523 641
- BÄTTIG SARAH ET AL: "Cassis and Green Tea: Spontaneous Release of Natural Aroma Compounds from [beta]-Alkylthioalkanones", vol. 104, no. 11, 1 November 2021 (2021-11-01), Hoboken, USA, XP093013525, ISSN: 0018-019X, Retrieved from the Internet <URL:https://onlinelibrary.wiley.com/doi/full-xml/10.1002/hlca.202100135> DOI: 10.1002/hlca.202100135
- ISHIKAWA YOUICHI, KISHI KIYOSHI: "Molecular orbital approach to odor molecules: Normal fatty acids and cyclamenaldehydes", INTERNATIONAL JOURNAL OF QUANTUM CHEMISTRY, WILEY, NEW YORK, NY, US, vol. 79, no. 2, 1 January 2000 (2000-01-01), US , pages 101 - 108, XP093117965, ISSN: 0020-7608, DOI: 10.1002/1097-461X(2000)79:2<101::AID-QUA5>3.0.CO;2-6

## Description

### Technical Field

The present invention relates to an aldehyde composition, a method for producing the same, and a fragrance composition containing the aldehyde composition.

### Background Art

Some 3-phenylpropanals are known to be useful as compounded fragrance ingredients.

For example, Non-Patent Document 1 discloses that phenylpropionaldehyde (3-phenylpropanal) having a hyacinth-like balsamic and green aroma, 3-(p-tert-butylphenyl)-2-methylpropanal (p-tert-butyl-α-methylhydrocinnamic aldehyde, Lilial) having a lily-of-the-valley-like aroma, 3-(p-isopropylphenyl)-2-methylpropanal (cyclamen aldehyde) having a cyclamen- and lily-of-the-valley-like aroma with a melon- and cucumber-like feeling, 3-(3,4-methylenedioxyphenyl)-2-methylpropanal (Helional) having a sweet heliotrope- and anise-like floral aroma, and the like are useful as compounded fragrance ingredients.

Patent Document 1 discloses a method for producing para-isobutyl dihydrocinnamic aldehyde having a cyclamen-like smell. It is disclosed that a product containing from 68 to 70% of a para isomer can be obtained through the following production method.

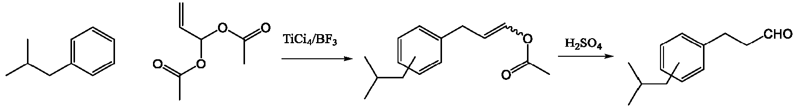

### Citation List

### Non-Patent Document

Non-Patent Document 1: Motoichi Indo, "Enlarged and Revised Edition, Synthetic Fragrance: Chemistry and Product Knowledge", The Chemical Daily Co., Ltd., 1996, pp. 211, 213 to 215, and 228

### Patent Document

Patent Document 1: JP S42-009135 B

### Summary of Invention

### Technical Problem

As described above, some 3-phenylpropanals have characteristic aroma. To enhance the value of products such as fragrance products, cosmetics, detergents, hygiene products, miscellaneous goods, pharmaceuticals, and foods, a new scent is required.

Patent Document 1 describes the above-described method for producing para-isobutyl dihydrocinnamic aldehyde (3-(4-isobutylphenyl)propanal) and a product fragrance composition containing 70 wt.% of a para isomer, but the relationship between the composition and the scent has not been studied in detail.

An object of the present invention is to provide an aldehyde composition having a novel scent and useful as a fragrance, and a fragrance composition.

### Solution to Problem

The inventors of the present invention have produced various aldehyde compositions and evaluated their aromas, and found that a specific aldehyde composition has an excellent scent and is excellent as an ingredient of a fragrance composition, thereby completing the present invention.

That is, the present invention is as follows.
[1] An aldehyde composition containing an aldehyde represented by Formula (1) described below and an aldehyde represented by Formula (2) described below, wherein a mass ratio [(1)/(2)] between the aldehyde represented by Formula (1) and the aldehyde represented by Formula (2) is from 97.00/3.00 to 99.999/0.001 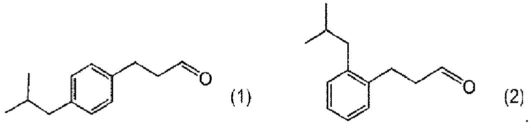
[2] The aldehyde composition according to [1], wherein the composition contains the aldehyde represented by Formula (1) described below and the aldehyde represented by Formula (2) described below, and the mass ratio [(1)/(2)] between the aldehyde represented by Formula (1) and the aldehyde represented by Formula (2) is from 97.00/3.00 to 99.90/0.10 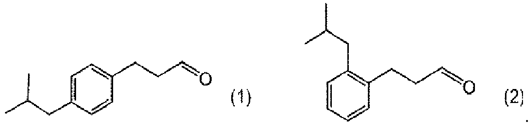
[3] The aldehyde composition according to [1] or [2], wherein the composition contains the aldehyde represented by Formula (1) described below and the aldehyde represented by Formula (2) described below, the mass ratio [(1)/(2)] between the aldehyde represented by Formula (1) and the aldehyde represented by Formula (2) is from 97.00/3.00 to 99.999/0.001, and a content of an ester represented by Formula (4) described below is 0.10 mass% or less with respect to a total amount of the aldehyde represented by Formula (1) and the aldehyde represented by Formula (2) 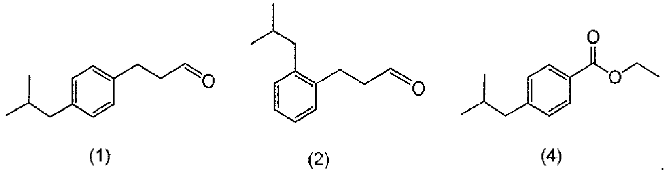
[4] The aldehyde composition according to [1] or [2], wherein the composition contains the aldehyde represented by Formula (1) described below, the aldehyde represented by Formula (2) described below, an ester represented by Formula (4) described below, the mass ratio [(1)/(2)] between the aldehyde represented by Formula (1) and the aldehyde represented by Formula (2) is from 97.00/3.00 to 99.999/0.001, and a content of the ester represented by Formula (4) is from 0.50 to 2.00 mass% or less with respect to a total amount of the aldehyde represented by Formula (1) and the aldehyde represented by Formula (2) 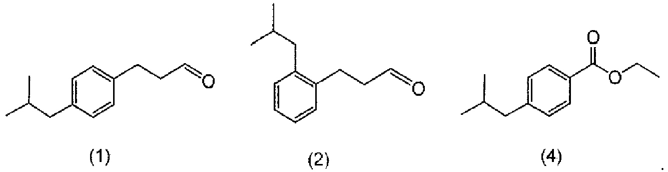
[5] The aldehyde composition according to any one of [1] to [4], wherein a total purity of the aldehydes represented by Formula (1) and Formula (2) is 97 mass% or more.
[6] The aldehyde composition according to any one of [1] to [5], wherein the composition includes an alcohol represented by Formula (3) described below in an amount from 0.01 to 0.90 mass% 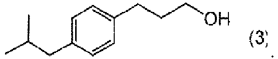
[7] The aldehyde composition according to any one of [1] to [6], wherein the composition includes a sulfonic acid ester represented by Formula (5) described below in an amount from 0.01 to 0.90 mass% 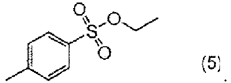
[8] A fragrance composition containing the aldehyde composition described in any one of [1] to [7].
[9] A method for producing the aldehyde composition described in any one of [1] to [7], the method including, in this order: a step of formylating isobutylbenzene with carbon monoxide under a superstrong acid condition to obtain 4-isobutylbenzaldehyde and 2-isobutylbenzaldehyde; a step of performing aldol condensation of 4-isobutylbenzaldehyde and 2-isobutylbenzaldehyde with acetaldehyde to obtain an aldehyde represented by Formula (6) described below and an aldehyde represented by Formula (7) described below; and a step of performing hydrogenation to obtain an aldehyde represented by Formula (1) described below and an aldehyde represented by Formula (2) described below 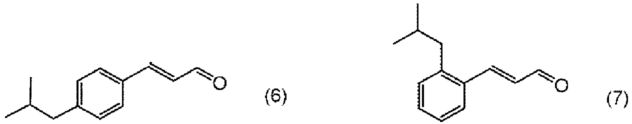 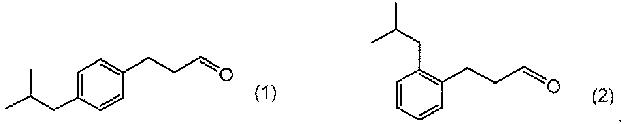
[10] A method for producing the aldehyde composition described in any one of [1] to [7], the method including, in this order: a step of formylating isobutylbenzene with carbon monoxide under a superstrong acid condition to obtain 4-isobutylbenzaldehyde and 2-isobutylbenzaldehyde; a step of acetalizing 4-isobutylbenzaldehyde and 2-isobutylbenzaldehyde in the presence of a para-toluenesulfonic acid catalyst;
   a step of causing acetals obtained to react with an alkyl vinyl ether under an acid catalyst; a step of hydrolyzing in the presence of an acid catalyst to obtain an aldehyde represented by Formula (6) described below and an aldehyde represented by Formula (7) described below; and a step of performing hydrogenation to obtain an aldehyde represented by Formula (1) described below and an aldehyde represented by Formula (2) described below

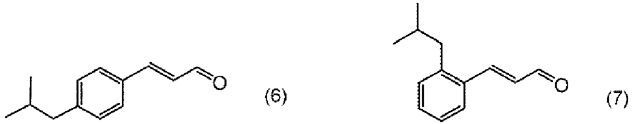

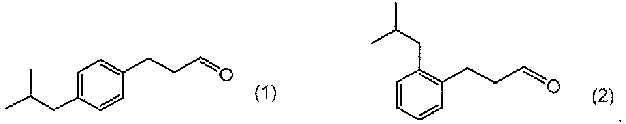

### Advantageous Effects of Invention

The present invention can provide an aldehyde composition useful as a fragrance since the composition has a scent excellent in balance of green, aldehyde, and floral. Further, a fragrance composition excellent in diffusibility can be provided by containing the aldehyde composition.

### Description of Embodiments

Hereinafter, in the present specification, the expression "from XX to YY" means "XX or more and YY or less".

### [Aldehyde Composition]

An aldehyde composition according to the present invention contains an aldehyde represented by Formula (1) described below and an aldehyde represented by Formula (2) described below, in which a mass ratio [(1)/(2)] between the aldehyde represented by Formula (1) to the aldehyde represented by Formula (2) is from 97.00/3.00 to 99.999/0.001.

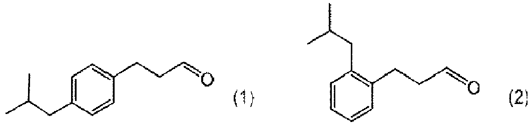

The aldehyde composition has a scent excellent in balance of green, aldehyde, and floral, and can impart diffusibility to a fragrance composition.

The aldehyde composition of the present invention contains the aldehyde represented by Formula (1) and the aldehyde represented by Formula (2), and these two components alone may be used as the components constituting the aldehyde composition of the present invention. However, in practice, by-products and raw materials produced when the aldehyde composition is produced may be contained.

Thus, the total purity of the aldehyde represented by Formula (1) and the aldehyde represented by Formula (2) in the aldehyde composition of the present invention is preferably 97 mass% or more, more preferably 97.5 mass% or more, and even more preferably 98 mass% or more. The upper limit of the total content is not limited, and may be 100 mass% or less, and the aldehyde composition of the present invention may be formed of only the aldehyde represented by Formula (1) and the aldehyde represented by Formula (2).

Hereinafter, preferred embodiments of the aldehyde composition of the present invention will be described.

### [Aldehyde Composition (First Embodiment)]

Among the aldehyde compositions of the present invention, an aldehyde composition that is a first embodiment as a preferred embodiment (hereinafter also referred to as first aldehyde composition) contains an aldehyde represented by Formula (1) described below and an aldehyde represented by Formula (2) described below, in which the mass ratio [(1)/(2)] between the aldehyde represented by Formula (1) and the aldehyde represented by Formula (2) is from 97.00/3.00 to 99.90/0.10.

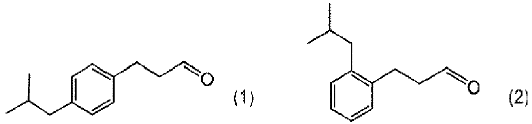

The mass ratio [(1)/(2)] between the aldehyde represented by Formula (1) and the aldehyde represented by Formula (2) in the first aldehyde composition is from 97.00/3.00 to 99.90/0.10, preferably 98.00/2.00 to 99.90/0.10, more preferably 98.50/1.50 to 99.90/0.10, even more preferably 99.00/1.00 to 99.80/0.20, and even more preferably 99.20/0.80 to 99.70/0.30.

The first aldehyde composition has a scent excellent in balance of green, aldehyde, and floral as described above, and further has a strong floral feeling, and thus is useful as a fragrance.

### [Aldehyde Composition (Second Embodiment)]

Among the aldehyde compositions of the present invention, an aldehyde composition of a second embodiment as a preferred embodiment (hereinafter also referred to as second aldehyde composition) contains an aldehyde represented by Formula (1) described below and an aldehyde represented by Formula (2) described below, in which the mass ratio [(1)/(2)] between the aldehyde represented by Formula (1) and the aldehyde represented by Formula (2) is 97.00/3.00 to 99.999/0.001, and a content of an ester represented by Formula (4) described below is 0.10 mass% or less with respect to the total amount of the aldehyde represented by Formula (1) and the aldehyde represented by Formula (2).

When the content of the ester represented by Formula (4) is 0.10 mass% or less with respect to the total amount of the aldehyde represented by Formula (1) and the aldehyde represented by Formula (2), the second aldehyde composition includes a case where the content of the ester represented by Formula (4) is 0 mass% with respect to the total amount of the aldehyde represented by Formula (1) and the aldehyde represented by Formula (2), that is, the ester represented by Formula (4) is not contained. However, the second aldehyde composition preferably contains the ester represented by Formula (4).

Thus, it is preferable that the second aldehyde composition contains the aldehyde represented by Formula (1) and the aldehyde represented by Formula (2), and the composition does not contain the ester represented by Formula (4) or contains the ester represented by Formula (4), in which the mass ratio [(1)/(2)] between the aldehyde represented by Formula (1) and the aldehyde represented by Formula (2) is from 97.00/3.00 to 99.999/0.001, and the content of the ester represented by Formula (4) is 0.10 mass% or less with respect to the total amount of the aldehyde represented by Formula (1) and the aldehyde represented by Formula (2). It is more preferable that the second aldehyde composition contains the aldehyde represented by Formula (1), the aldehyde represented by Formula (2), and the ester represented by Formula (4), in which the mass ratio [(1)/(2)] between the aldehyde represented by Formula (1) and the aldehyde represented by Formula (2) is from 97.00/3.00 to 99.999/0.001, and the content of the ester represented by Formula (4) is 0.10 mass% or less with respect to the total amount of the aldehyde represented by Formula (1) and the aldehyde represented by Formula (2).

The second aldehyde composition may be contained in the first aldehyde composition. That is, an aldehyde composition that is the second aldehyde composition and is the first aldehyde composition is also included in the aldehyde composition of the present invention.

The aldehyde composition that is the second aldehyde composition and is the first aldehyde composition contains the aldehyde represented by Formula (1) and the aldehyde represented by Formula (2), in which the mass ratio [(1)/(2)] between the aldehyde represented by Formula (1) and the aldehyde represented by Formula (2) is from 97.00/3.00 to 99.90/0.10, and the content of the ester represented by Formula (4) is 0.10 mass% or less with respect to the total amount of the aldehyde represented by Formula (1) and the aldehyde represented by Formula (2).

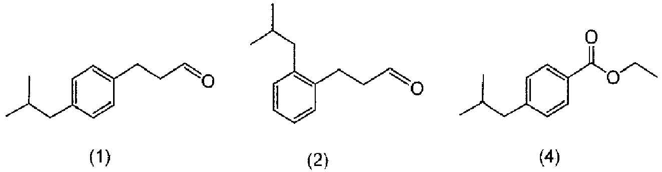

The mass ratio [(1)/(2)] between the aldehyde represented by Formula (1) and the aldehyde represented by Formula (2) in the second aldehyde composition is from 97.00/3.00 to 99.999/0.001, preferably from 98.00/2.00 to 99.999/0.001, more preferably from 98.50/1.50 to 99.999/0.001, even more preferably from 99.50/0.50 to 99.998/0.002, even more preferably from 99.90/0.10 to 99.998/0.002, and even more preferably from 99.96/0.04 to 99.997/0.003.

The content of the ester represented by Formula (4) in the second aldehyde composition is 0.10 mass% or less, preferably 0.05 mass% or less, and more preferably 0.02 mass% or less, with respect to the total amount of the aldehyde represented by Formula (1) and the aldehyde represented by Formula (2). The lower limit is not limited, and the second aldehyde composition includes a case where the ester represented by Formula (4) is not contained, but the lower limit is preferably 0.0001 mass% or more, more preferably 0.001 mass% or more, and even more preferably 0.01 mass% or more.

The second aldehyde composition has a scent excellent in balance of green, aldehyde, and floral as described above, and further has a strong aldehyde feeling, and thus is useful as a fragrance. The ester represented by Formula (4) may be obtained as a by-product in the production of the aldehyde represented by Formula (1) and the aldehyde represented by Formula (2). **In** such a case, the second aldehyde composition can be obtained without adding the ester represented by Formula (4), but the second aldehyde composition may be obtained by adding the ester represented by Formula (4) to an aldehyde composition not containing the ester represented by Formula (4). When the ester represented by Formula (4) obtained as a by-product in the production of the aldehyde represented by Formula (1) and the aldehyde represented by Formula (2) is more than 0.10 mass% with respect to the total amount of the aldehyde represented by Formula (1) and the aldehyde represented by Formula (2), the ester may be reduced to 0.10 mass% or less through distillation purification or chromatography.

### [Aldehyde Composition (Third Embodiment)]

Among the aldehyde compositions of the present invention, an aldehyde composition of a third embodiment as a preferred embodiment (hereinafter also referred to as third aldehyde composition) contains an aldehyde represented by Formula (1) described below, an aldehyde represented by Formula (2) described below, and an ester represented by Formula (4) described below, in which the mass ratio [(1)/(2)] between the aldehyde represented by Formula (1) and the aldehyde represented by Formula (2) is 97.00/3.00 to 99.999/0.001, and a content of an ester represented by Formula (4) described below is from 0.50 to 2.00 mass% with respect to the total amount of the aldehyde represented by Formula (1) and the aldehyde represented by Formula (2).

The third aldehyde composition may be contained in the first aldehyde composition. That is, an aldehyde composition that is the third aldehyde composition and is the first aldehyde composition is also included in the aldehyde composition of the present invention.

The aldehyde composition that is the third aldehyde composition and is the first aldehyde composition contains the aldehyde represented by Formula (1) and the aldehyde represented by Formula (2), in which the mass ratio [(1)/(2)] between the aldehyde represented by Formula (1) and the aldehyde represented by Formula (2) is from 97.00/3.00 to 99.90/0.10, and the content of the ester represented by Formula (4) is from 0.50 to 2.00 mass% with respect to the total amount of the aldehyde represented by Formula (1) and the aldehyde represented by Formula (2).

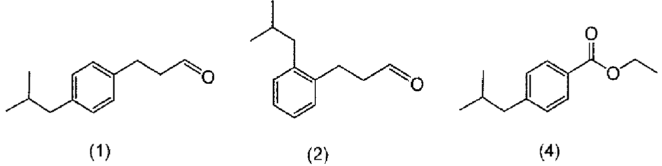

The mass ratio [(1)/(2)] between the aldehyde represented by Formula (1) and the aldehyde represented by Formula (2) in the third aldehyde composition is from 97.00/3.00 to 99.999/0.001, preferably from 98.00/2.00 to 99.999/0.001, more preferably from 98.50/1.50 to 99.999/0.001, even more preferably from 99.00/1.00 to 99.995/0.005, even more preferably from 99.90/0.10 to 99.99/0.01, and even more preferably from 99.92/0.08 to 99.98/0.02.

The content of the ester represented by Formula (4) in the third aldehyde composition is from 0.50 to 2.00 mass%, preferably from 0.50 to 1.00 mass%, and more preferably from 0.50 to 0.80 mass%, with respect to the total amount of the aldehyde represented by Formula (1) and the aldehyde represented by Formula (2).

The third aldehyde composition has a scent excellent in balance of green, aldehyde, and floral as described above, and further has a strong powdery feeling, and thus is useful as a fragrance. The ester represented by Formula (4) may be obtained as a by-product in the production of the aldehyde represented by Formula (1) and the aldehyde represented by Formula (2). In such a case, the third aldehyde composition can be obtained without adding the ester represented by Formula (4), but the third aldehyde composition may be obtained by adding the ester represented by Formula (4) to an aldehyde composition not containing the ester represented by Formula (4).

### [Additional Component]

The aldehyde composition of the present invention contains the aldehyde represented by Formula (1) and the aldehyde represented by Formula (2), in which the mass ratio [(1)/(2)] between the aldehyde represented Formula (1) and the aldehyde represented by Formula (2) is from 97.00/3.00 to 99.999/0.001, and it may contain the ester represented by Formula (4). The aldehyde composition of the present invention may further contain additional components described below, and by containing these components, it is possible to have variations in its scent when used as a fragrance, which is preferable.

The additional components described in this section are common to the first aldehyde composition, the second aldehyde composition, and the third aldehyde composition.

The aldehyde composition of the present invention may contain an alcohol represented by Formula (3) described below in an amount from 0.01 to 0.90 mass%.

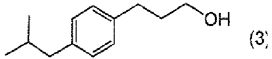

The content of the alcohol represented by Formula (3) in the aldehyde composition of the present invention is preferably from 0.01 to 0.90 mass%, more preferably from 0.01 to 0.50 mass%, and even more preferably from 0.01 to 0.30 mass%. The alcohol represented by Formula (3) may be obtained as a by-product in the production of the aldehyde represented by Formula (1) and the aldehyde represented by Formula (2), but the alcohol may be added to an aldehyde composition not containing the alcohol represented by Formula (3).

The aldehyde composition of the present invention may contain a sulfonic acid ester represented by Formula (5) described below in an amount from 0.01 to 0.90 mass%.

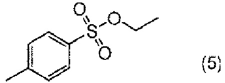

The content of the ester represented by Formula (5) in the aldehyde composition of the present invention is preferably from 0.01 to 0.90 mass%, more preferably from 0.01 to 0.50 mass%, and even more preferably from 0.01 to 0.30 mass%. The ester represented by Formula (5) may be obtained as a by-product in the production of the aldehyde represented by Formula (1) and the aldehyde represented by Formula (2), but the ester may be added to an aldehyde composition not containing the ester represented by Formula (5).

The aldehyde composition of the present invention has the above-described composition and has a scent excellent in balance of green, aldehyde, and floral, and thus is useful as a fragrance.

The aldehyde composition is also useful as an ingredient of a fragrance composition, can be used as an aromatic component of various products, and can impart diffusibility to the fragrance composition.

### [Fragrance Composition]

A fragrance composition of the present invention contains the aldehyde composition.

That is, the fragrance composition of the present invention contains the aldehyde composition containing an aldehyde represented by Formula (1) described below and an aldehyde represented by Formula (2) described below, in which the mass ratio [(1)/(2)] between the aldehyde represented by Formula (1) and the aldehyde represented by Formula (2) is from 97.00/3.00 to 99.999/0.001.

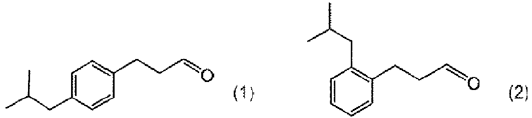

The fragrance composition of the present invention is excellent in diffusibility while having a scent excellent in balance of green, aldehyde, and floral of the aldehyde composition. Thus, it is useful as an aromatic component of various products.

As a preferred embodiment, the fragrance composition of the present invention contains the first aldehyde composition.

That is, a preferred fragrance composition of the present invention contains the aldehyde composition containing an aldehyde represented by Formula (1) described below and an aldehyde represented by Formula (2) described below, in which the mass ratio [(1)/(2)] between the aldehyde represented by Formula (1) and the aldehyde represented by Formula (2) is from 97.00/3.00 to 99.90/0.10.

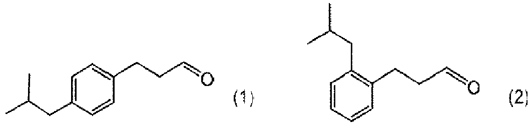

The fragrance composition of the present invention containing the first aldehyde composition is excellent in diffusibility while having a scent excellent in balance of green, aldehyde, and floral of the aldehyde composition and a stronger floral feeling. Thus, it is useful as an aromatic component of various products.

As a preferred embodiment, the fragrance composition of the present invention contains the second aldehyde composition.

That is, a preferred fragrance composition of the present invention contains an aldehyde composition containing the aldehyde represented by Formula (1) and the aldehyde represented by Formula (2), in which the mass ratio [(1)/(2)] between the aldehyde represented by Formula (1) and the aldehyde represented by Formula (2) is from 97.00/3.00 to 99.999/0.001, and the content of the ester represented by Formula (4) is 0.10 mass% or less with respect to the total amount of the aldehyde represented by Formula (1) and the aldehyde represented by Formula (2).

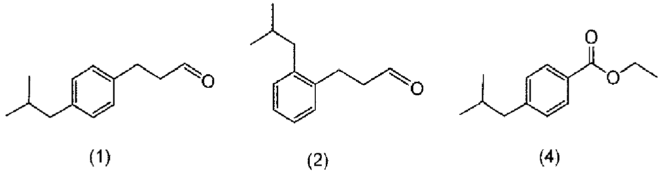

The fragrance composition of the present invention containing the second aldehyde composition is excellent in diffusibility while having a scent excellent in balance of green, aldehyde, and floral of the aldehyde composition, a stronger floral feeling, and further, a stronger aldehyde feeling. Thus, it is useful as an aromatic component of various products.

As a preferred embodiment, the fragrance composition of the present invention contains the third aldehyde composition.

That is, a preferred fragrance composition of the present invention contains an aldehyde composition containing the aldehyde represented by Formula (1), the aldehyde represented by Formula (2), and the ester represented by Formula (4), in which the mass ratio [(1)/(2)] between the aldehyde represented by Formula (1) and the aldehyde represented by Formula (2) is from 97.00/3.00 to 99.999/0.001, and the content of the ester represented by Formula (4) is from 0.50 to 2.00 mass% with respect to the total amount of the aldehyde represented by Formula (1) and the aldehyde represented by Formula (2).

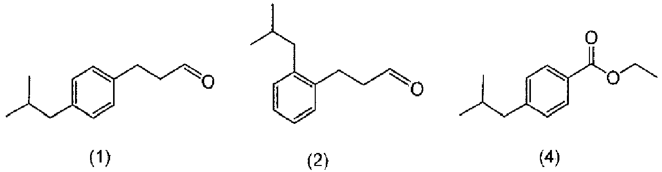

The fragrance composition of the present invention containing the third aldehyde composition is excellent in diffusibility while having a scent excellent in balance of green, aldehyde, and floral of the aldehyde composition, a stronger floral feeling, and further, a powdery feeling. Thus, it is useful as an aromatic component of various products.

The content of the aldehyde composition in the fragrance composition of the present invention is to be appropriately adjusted according to the type of fragrance composition, the type of target aroma, the intensity of the aroma, and the like and is preferably from 0.01 to 90 mass% and more preferably from 0.1 to 50 mass%.

Examples of additional components other than the aldehyde composition contained in the fragrance composition containing the aldehyde composition include a fragrance other than the aldehyde represented by Formula (1) or the aldehyde represented by Formula (2), a surfactant, a solvent, an antioxidant, and a colorant. At least one selected from the group consisting of a fragrance other than the aldehyde represented by Formula (1) or the aldehyde represented by Formula (2), a surfactant, a solvent, an antioxidant, and a colorant is preferable, and at least one selected from the group consisting of a fragrance other than the aldehyde represented by Formula (1) or the aldehyde represented by Formula (2), and a solvent is more preferable.

Containing a fragrance other than the aldehyde represented by Formula (1) or the aldehyde represented by Formula (2) can adjust a scent according to a target product. In addition, the inclusion of a solvent allows for easy dissolution in and impregnation of a target product, making it possible to adjust the intensity of the scent or the lasting property of the scent.

The fragrance other than the aldehyde represented by Formula (1) or the aldehyde represented by Formula (2) is not limited as long as it is a known fragrance component, and a wide range of fragrances can be used. For example, one, or two or more of the following fragrances can be selected and used at any mixing ratio.

Examples of the fragrance other than the aldehyde represented by Formula (1) or the aldehyde represented by Formula (2) include hydrocarbons, alcohols, phenols, esters, aldehydes, ketones, acetals, ketals, ethers, nitriles, lactones, natural essential oils, and natural extracts.

Examples of hydrocarbons include limonene, α-pinene, β-pinene, terpinene, cedrene, longifolene, and valencene.

Examples of alcohols include linalool, citronellol, geraniol, nerol, terpineol, dihydromyrcenol, ethyllinalool, farnesol, nerolidol, cis-3-hexenol, cedrol, menthol, borneol, β-phenylethyl alcohol, benzyl alcohol, phenyl hexanol, 2,2,6-trimethylcyclohexyl-3-hexanol, 1-(2-t-butylcyclohexyloxy)-2-butanol, 4-isopropylcyclohexane methanol, 4-t-butylcyclohexanol, 4-methyl-2-(2-methylpropyl)tetrahydro-2H-pyran-4-ol, 2-methyl-4-(2,2,3-trimethyl-3-cyclopenten-1-yl)-2-buten-1-ol, 2-ethyl-4-(2,2,3-trimethyl-3-cyclopenten-1-yl)-2-buten-1-ol, isocamphylcyclohexanol, and 3,7-dimethyl-7-methoxyoctan-2-ol.

Examples of phenols include eugenol, thymol, and vanillin.

Examples of esters include linalyl formate, citronellyl formate, geranyl formate, n-hexyl acetate, cis-3-hexenyl acetate, linalyl acetate, citronellyl acetate, geranyl acetate, nellyl acetate, terpinyl acetate, nopil acetate, bornyl acetate, isobornyl acetate, o-t-butylcyclohexyl acetate, p-t-butylcyclohexyl acetate, tricyclodecenyl acetate, benzyl acetate, styralyl acetate, cinnamyl acetate, dimethylbenzyl carbinyl acetate, 3-pentyltetrahydropyran-4-yl acetate, citronellyl propionate, tricyclodecenyl propionate, allylcyclohexyl propionate, ethyl-2-cyclohexyl propionate, benzyl propionate, citronellyl butyrate, dimethylbenzylcarbinyl n-butyrate, tricyclodecenyl isobutyrate, methyl-2-nonenoate, methyl benzoate, benzyl benzoate, methyl cinnamate, methyl salicylate, n-hexyl salicylate, cis-3-hexenyl salicylate, geranyl tiglate, cis-3-hexenyl tiglate, methyl jasmonate, methyldihydro jasmonate, methyl-2,4-dihydroxy-3,6-dimethyl benzoate, ethylmethylphenyl glycidate, methyl anthranilate, and fruitate.

Examples of aldehydes include n-octanal, n-decanal, n-dodecanal, 2-methylundecanal, 10-undecenal, citronellal, citral, hydroxycitronellal, dimethyl tetrahydrobenzaldehyde, 4-(4-hydroxy-4-methylpentyl)-3-cyclohexene-1-carboaldehyde, 2-cyclohexyl propanal, p-t-butyl-α-methylhydrocinnamic aldehyde, p-t-butylhydrocinnamic aldehyde, p-isopropyl-α-methylhydrocinnamic aldehyde, p-ethyl-α,α-dimethylhydrocinnamic aldehyde, α-amylcinnamic aldehyde, α-hexylcinnamic aldehyde, piperonal, and α-methyl-3,4-methylenedioxyhydrocinnamic aldehyde.

Examples of ketones include methylheptenone, 4-methylene-3,5,6,6-tetramethyl-2-heptanone, amylcyclopentanone, 3-methyl-2-(cis-2-penten-1-yl)-2-cyclopenten-1-one, methylcyclopentenolone, rose ketone, γ-methylionone, α-ionone, carbone, menthone, camphor, nootkatone, benzylacetone, anysilacetone, methyl-β-naphthylketone, 2,5-dimethyl-4-hydroxy-3(2H)-furanone, maltol, 7-acetyl-1,2,3,4,5,6,7,8-octahydro-1,1,6,7-tetramethyl naphthalene, muscone, civetone, cyclopentadecanone, and cyclohaxedecenone.

Examples of acetals and ketals include acetaldehyde ethylphenylpropyl acetal, citraldiethyl acetal, phenylacetaldehyde glycerin acetal, and ethylacetoacetate ethylene glycol ketal.

Examples of ethers include anetol, β-naphthylmethyl ether, β-naphthylethyl ether, limonene oxide, rose oxide, 1,8-cineol, racemic or optically active dodecahydro-3a,6,6,9a-tetramethylnaphtho[2,1-b]furane.

Examples of nitriles include citronellyl nitrile.

Examples of lactones include γ-nonalactone, γ-undecalactone, σ-decalactone, γ-jasmolactone, cumarin, cyclopentadecanolide, cyclohexadecanolide, ambrettolide, ethylene brassylate, and 11-oxahexadecanolide.

Examples of natural essential oils and natural extracts include those of orange, lemon, bergamot, mandarin, peppermint, spearmint, lavender, chamomile, rosemary, eucalyptus, sage, basil, rose, geranium, jasmine, ylang-ylang, anise, clove, ginger, nutmeg, cardamom, cedar, Japanese cypress, sandalwood, vetiver, patchouli, and labdanum.

Examples of the solvent include dipropylene glycol, diethyl phthalate, ethylene glycol, propylene glycol, methyl myristate, and triethyl citrate.

Examples of the surfactant include polyoxyethylene lauryl sulfate ether.

The fragrance composition containing the aldehyde composition can be used as an aromatic component for various products.

The products in which the fragrance composition can be used include: fragrance products, such as perfumes and colognes; cosmetics for hair, such as shampoos, conditioners, hair tonics, hair creams, mousses, gels, pomades, and sprays; cosmetics for skin, such as face lotions, serums, creams, emulsions, facial masks, foundations, face powders, lipsticks, and various types of makeup; various detergents for health and sanitation, such as dish detergents, laundry detergents, softeners, disinfectant detergents, odor eliminating detergents, indoor fragrances, furniture care products, glass cleaners, furniture cleaners, floor cleaners, disinfectants, insecticides, bleaches, germicides, and repellents; quasi-drugs, such as toothpaste, mouthwash, bath salts, antiperspirants, and perm solutions; miscellaneous goods such as toilet paper and tissue paper; pharmaceuticals; and food products.

The amount of the fragrance composition added to the products described above is not limited and can be selected depending on the type, nature, and sensory effect of the product to be perfumed. For example, the amount of the fragrance composition added to the products is preferably 0.00001 mass% or greater, more preferably 0.0001 mass% or greater, and even more preferably 0.001 mass% or greater. Also, the amount of the fragrance composition added to the products is preferably 80 mass% or less, more preferably 60 mass% or less, and even more preferably 40 mass% or less.

When the fragrance composition is used as an aromatic oil, perfume, or the like, the amount of the fragrance composition added to the products may be 80 mass% or greater, or may be 100 mass%.

### [Method for Producing Aldehyde Composition]

The method for producing the aldehyde composition of the present invention is not limited as long as the composition contains the aldehyde represented by Formula (1) and the aldehyde represented by Formula (2), in which the mass ratio [(1)/(2)] thereof is from 97.00/3.00 to 99.999/0.001 as described above, but it is preferably obtained by the following production method.

As preferred methods for producing the aldehyde composition of the present invention, two methods are described below.

### (First production method)

That is, a first production method is a method for producing an aldehyde composition including, in this order, a step of formylating isobutylbenzene with carbon monoxide under a superstrong acid condition to obtain 4-isobutylbenzaldehyde and 2-isobutylbenzaldehyde,
a step of performing aldol condensation of 4-isobutylbenzaldehyde and 2-isobutylbenzaldehyde with acetaldehyde to obtain an aldehyde represented by Formula (6) described below and an aldehyde represented by Formula (7) described below, and
a step of performing hydrogenation to obtain an aldehyde represented by Formula (1) described below and an aldehyde represented by Formula (2) described below.

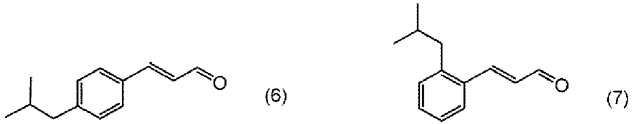

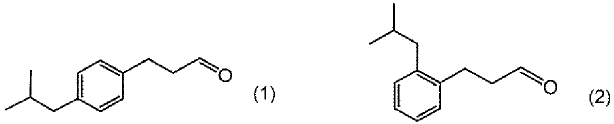

### (Second production method)

A second production method is a method for producing an aldehyde composition including, in this order, a step of formylating isobutylbenzene with carbon monoxide under a superstrong acid condition to obtain 4-isobutylbenzaldehyde and 2-isobutylbenzaldehyde,
a step of acetalizing 4-isobutylbenzaldehyde and 2-isobutylbenzaldehyde in the presence of a para-toluenesulfonic acid catalyst,
a step of causing the resulting acetals to react with an alkyl vinyl ether under an acid catalyst,
a step of hydrolyzing in the presence of an acid catalyst to obtain an aldehyde represented by Formula (6) described below and an aldehyde represented by Formula (7) described below, and
a step of performing hydrogenation to obtain an aldehyde represented by Formula (1) described below and an aldehyde represented by Formula (2) described below.

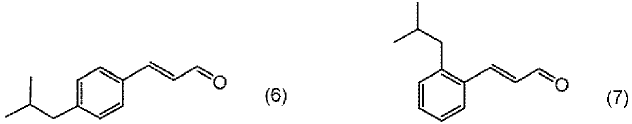

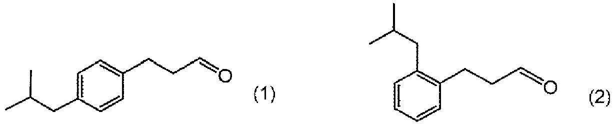

Each production method will be described below.

### <First Production Method>

That is, a first production method is a method for producing an aldehyde composition including, in this order, a step of formylating isobutylbenzene with carbon monoxide under a superstrong acid condition to obtain 4-isobutylbenzaldehyde and 2-isobutylbenzaldehyde,
a step of performing aldol condensation of 4-isobutylbenzaldehyde and 2-isobutylbenzaldehyde with acetaldehyde to obtain an aldehyde represented by Formula (6) described below and an aldehyde represented by Formula (7) described below, and
a step of performing hydrogenation to obtain an aldehyde represented by Formula (1) described below and an aldehyde represented by Formula (2) described below.

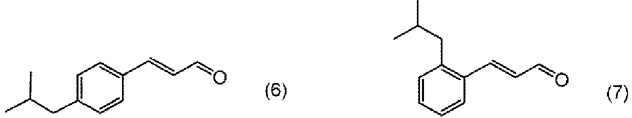

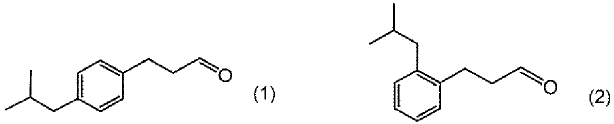

### (Formylation step)

The first step in the first production method is a step of formylating isobutylbenzene with carbon monoxide under a superstrong acid condition to obtain 4-isobutylbenzaldehyde and 2-isobutylbenzaldehyde. "Under a superstrong acid condition" means that a superstrong acid catalyst is used for the reaction.

The superstrong acid catalyst is preferably a combination of a Bronsted acid and a Lewis acid.

The Bronsted acid is not limited, but hydrogen fluoride, hydrogen chloride, and hydrogen bromide are preferable, and hydrogen fluoride is more preferable.

The Lewis acid is not limited, but boron trifluoride and aluminum chloride are preferable, and boron trifluoride is more preferable.

From the viewpoints of recovery and reuse of the catalyst and positional selectivity of the formyl group, a combination of hydrogen fluoride and boron trifluoride is even more preferable.

When hydrogen fluoride is used as the Bronsted acid, hydrogen fluoride also has a function as a solvent for the reaction. The hydrogen fluoride is preferably substantially anhydrous hydrogen fluoride from the perspective of the reactivity. The phrase "substantially anhydrous" means that the water content is 5 mass% or less, preferably 1 mass% or less, and more preferably 0.1 mass% or less.

The molar ratio of hydrogen fluoride to isobutylbenzene [hydrogen fluoride (mol)/isobutylbenzene (mol)] is preferably 3.0 or more, more preferably 5.0 or more, even more preferably 10.0 or more from the viewpoint of reactivity with carbon monoxide and suppression of side reactions, and is preferably 30.0 or less, more preferably 20.0 or less, even more preferably 15.0 or less from the viewpoint of economic efficiency and production efficiency.

When boron trifluoride is used as the Lewis acid, the molar ratio of boron trifluoride to isobutylbenzene [boron trifluoride (mol)/isobutylbenzene (mol)] is preferably 1.0 or more, more preferably 2.0 or more, and is preferably 4.0 or less, more preferably 3.0 or less, from the viewpoint of reactivity with carbon monoxide and suppression of side reactions.

The reaction temperature is preferably -50°C or more, more preferably -40°C or more, even more preferably -30°C or more, and is preferably 30°C or less, more preferably 0°C or less, even more preferably -10°C or less, from the viewpoint of improving the reactivity, suppressing side reactions, and improving the selectivity of the position at which the formyl group is introduced.

The reaction between isobutylbenzene and carbon monoxide is preferably performed under pressure. The pressure during the reaction is preferably 1.0 MPaG or more, more preferably 1.5 MPaG or more, and even more preferably 1.8 MPaG or more, and is preferably 3.0 MPaG or less, more preferably 2.5 MPaG or less, and even more preferably 2.2 MPaG or less, as a partial pressure of carbon monoxide, from the viewpoint of improving the reactivity and suppressing side reactions.

In the reaction (formylation reaction), the reaction time is not particularly limited, and is preferably 10 minutes or more, more preferably 20 minutes or more, and even more preferably 30 minutes or more, and is preferably 24 hours or less, more preferably 12 hours or less, and even more preferably 5 hours or less, from the viewpoint of sufficiently advancing the reaction, suppressing side reactions and decomposition of products, and efficient production.

The reaction may be performed in the presence of a solvent. The solvent to be used is not particularly limited as long as the solvent has good solubility of the reaction raw material and is inert to acid catalysts such as hydrogen fluoride and boron trifluoride. Examples thereof include saturated aliphatic hydrocarbons such as hexane, heptane, and decane, and halogenated aliphatic hydrocarbons such as chloroform, methylene chloride, and dichloroethane. One of these solvents may be used alone, or two or more of these may be used in combination. The amount of solvent to be used is not particularly limited, and is only required to be selected as appropriate from the perspective of the uniformity of the reaction, reaction rate, and solvent removal. When hydrogen fluoride is used in this step, the solvent does not have to be used because hydrogen fluoride also functions as a solvent.

The above reaction may be any method such as a batch type, a semi-batch type, a continuous type, and the like, but is preferably a continuous type, from the perspective that the catalyst can be recovered and recycled and the perspective of production efficiency.

The apparatus to be used in the production method is a reaction apparatus capable of sufficiently mixing the liquid phase and the gas phase while adjusting the temperature under pressure.

For example, in the continuous type, first, hydrogen fluoride and boron trifluoride are placed into a reactor equipped with a stirrer, the contents are stirred, the liquid temperature is set to a suitable temperature, and the temperature is kept constant. Then, the pressure is raised to a suitable reaction pressure with carbon monoxide, and carbon monoxide is allowed to be supplied so that the pressure is kept constant. Thereafter, a semi-batch type reaction in which isobutylbenzene dissolved in a solvent as necessary is supplied is performed. Further subsequently, supply of hydrogen fluoride, boron trifluoride, and isobutylbenzene dissolved in a solvent as necessary is started, and the reaction product solution is continuously extracted.

Hydrogen fluoride and boron trifluoride are removed from the obtained reaction product solution containing 4-isobutylbenzaldehyde and 2-isobutylbenzaldehyde, and as necessary, the reaction product solution is purified by distillation or extraction to obtain the target 4-isobutylbenzaldehyde and 2-isobutylbenzaldehyde.

When 2-isobutylbenzaldehyde is contained in the obtained 4-isobutylbenzaldehyde, further purification may be performed to obtain 4-isobutylbenzaldehyde of higher purity and 2-isobutylbenzaldehyde of higher purity, or a mixture thereof may be used as a raw material in the next step as it is.

In particular, when the mass ratio between 4-isobutylbenzaldehyde and 2-isobutylbenzaldehyde [4-isobutylbenzaldehyde/2-isobutylbenzaldehyde] is from 97.00/3.00 for 99.999/0.001, use of the compound as it is as a raw material allows the mass ratio of the final product to be in the range (mass ratio between aldehyde represented by Formula (1) and aldehyde represented by Formula (2) [(1)/(2)]) of from 97.00/3.00 to 99.999/0.001, which is preferable.

The mass ratio between 4-isobutylbenzaldehyde and 2-isobutylbenzaldehyde [4-isobutylbenzaldehyde/2-isobutylbenzaldehyde] is preferably adjusted depending on the target scent. For example, when the purpose is to obtain the first aldehyde composition, the mass ratio is preferably from 97.00/3.00 to 99.90/0.10, more preferably from 98.00/2.00 to 99.90/0.10, even more preferably from 98.50/1.50 to 99.90/0.10, even more preferably from 99.00/1.00 to 99.80/0.20, and even more preferably from 99.20/0.80 to 99.70/0.30. The mass ratio is preferably from 97.00/3.00 to 99.999/0.001, more preferably from 98.00/2.00 to 99.999/0.001, even more preferably from 98.50/1.50 to 99.999/0.001, even more preferably from 99.50/0.50 to 99.998/0.002, even more preferably from 99.90/0.10 to 99.998/0.002, and even more preferably from 99.96/0.04 to 99.997/0.003, when it is intended to obtain the second aldehyde composition. The mass ratio is preferably from 97.00/3.00 to 99.999/0.001, more preferably from 98.00/2.00 to 99.999/0.001, even more preferably from 98.50/1.50 to 99.999/0.001, even more preferably from 99.00/1.00 to 99.995/0.005, even more preferably from 99.90/0.10 to 99.99/0.01, and even more preferably from 99.92/0.08 to 99.98/0.02, when it is intended to obtain the third aldehyde composition.

The mass ratio of positional isomers in the final product can be adjusted not only in this step but also in any step in the production method. Specifically, the ratio of positional isomers of the raw materials in each step corresponding to the target aldehyde may be adjusted.

### (Cinnamic aldehyde synthesis step using aldol condensation)

In the first production method, the next step is a step of performing aldol condensation of 4-isobutylbenzaldehyde and 2-isobutylbenzaldehyde obtained in the previous step with acetaldehyde to obtain the aldehyde represented by Formula (6) and the aldehyde represented by Formula (7) (isobutyl cinnamic aldehyde).

In the aldol condensation reaction in this step, a basic compound is preferably used as a catalyst.

Examples of the basic compound used as a catalyst include sodium hydroxide, potassium hydroxide, sodium bicarbonate, and mixtures of these. The amount of the basic compound is preferably 0.05 mol or more, more preferably 0.1 mol or more, and even more preferably 0.2 mol or more, and preferably 3 mol or less, more preferably 1 mol or less, and even more preferably 0.5 mol or less with respect to 1 mol of isobutylbenzaldehyde as a raw material.

The amount of acetaldehyde to be added is preferably 0.5 mol or more, more preferably 0.8 mol or more, and is preferably 1.5 mol or less, more preferably 1.1 mol or less with respect to 1 mol of 4-isobutylbenzaldehyde as a raw material. Acetaldehyde is preferably added sequentially or continuously over time, and for example, is preferably added dropwise.

The aldol condensation reaction in this step is preferably performed in a solvent. Examples of the solvent include various water-miscible organic solvents. Specifically, preferable examples thereof include alcohols, such as methanol, ethanol, 1-propanol, 2-propanol, tert-butanol, allyl alcohol, ethylene glycol, propylene glycol, and diethylene glycol, and more preferable examples include methanol, ethanol, 1-propanol, 2-propanol, tert-butanol, ethylene glycol, propylene glycol, and diethylene glycol.

The reaction temperature in the aldol condensation reaction in this step is not particularly limited but is preferably 0°C or more, and more preferably 10°C or more from the viewpoint of the reaction rate, and preferably 50°C or less, more preferably 40°C or less, and even more preferably 30°C or less from the viewpoint of preventing a side reaction.

The reaction time is not particularly limited and is to be a time in which the condensation sufficiently takes place. The reaction time is preferably 10 minutes or more, more preferably 30 minutes or more, and even more preferably 1 hour or more, and is preferably 24 hours or less, more preferably 12 hours or less, even more preferably 6 hours or less, and still more preferably 4 hours or less.

The reaction is to be stopped by neutralization; for example, the reaction can be stopped by adding an acid, such as acetic acid.

The method for isolating the aldehyde represented by Formula (6) and the aldehyde represented by Formula (7) from the solution after completion of the reaction is not particularly limited, and it may be performed by appropriately combining liquid separation, extraction operation, and distillation purification.

For example, a low-polar or non-polar organic solvent is added to the solution after completion of the reaction to transfer the aldehyde mixture to the oil phase, and the resulting oil phase is dried, for example, with magnesium sulfate. The filtrate obtained by filtration is then concentrated, and further, the concentrate is purified by distillation, whereby the aldehydes can be isolated.

### (Hydrogenation step)

In the first production method, the next step is a step of performing hydrogenation to obtain the aldehyde represented by Formula (1) and the aldehyde represented by Formula (2).

This hydrogenation step is a step of hydrogenating the aldehyde represented by Formula (6) and the aldehyde represented by Formula (7) (isobutyl cinnamic aldehyde) obtained in the previous step to obtain a target aldehyde composition containing the aldehyde represented by Formula (1) and the aldehyde represented by Formula (2).

The hydrogenation method is not particularly limited but can be performed by a known method using a hydrogenation catalyst.

The hydrogenation catalyst is not particularly limited, and a known catalyst can be used. The hydrogenation catalyst is not particularly limited, and a known catalyst can be used, including, for example, a supported heterogeneous hydrogenation catalyst in which a metal, such as Ni, Pt, Pd, or Ru, is supported on carbon, silica, alumina, diatomaceous earth, or the like; what is called a Ziegler-type hydrogenation catalyst prepared using a transition metal salt, such as an organic acid salt and an acetylacetone salt of Ni, Co, Fe, Cr, or the like, and a reducing agent, such as organoaluminum; and a homogeneous hydrogenation catalyst of what is called an organometallic complex or the like, such as an organometallic compound of Ti, Ru, Rh, Zr, or the like.

The temperature of the hydrogenation reaction in this step is preferably 0°C or more, more preferably 10°C or more, and even more preferably 20°C or more, and is preferably 150°C or less and more preferably 100°C or less from the viewpoints of the reactivity and suppressing side reactions.

The pressure of hydrogen used in the hydrogenation reaction is preferably 0.01 MPaG or more, more preferably 0.03 MPaG or more, and even more preferably 0.05 MPaG or more, and is preferably 10 MPaG or less, more preferably 3 MPaG or less, even more preferably 1 MPaG or less, and still more preferably 0.5 MPaG or less.

The reaction time is not particularly limited but is preferably 3 minutes or more, more preferably 10 minutes or more, and even more preferably 30 minutes or more, and is preferably 24 hours or less, more preferably 12 hours or less, and even more preferably 8 hours or less.

The hydrogenation reaction may be performed in the presence of a solvent. The solvent to be used is not particularly limited as long as the hydrogenation reaction is not inhibited but is exemplified by hydrocarbon solvents including: aliphatic hydrocarbons, such as pentane, hexane, isopentane, heptane, octane, and isooctane; aliphatic hydrocarbons, such as cyclopentane, methylcyclopentane, cyclohexane, methylcyclohexane, and ethylcyclohexane; and aromatic hydrocarbons, such as benzene, toluene, ethylbenzene, and xylene. One of these may be used individually, or two or more of these may be used in combination.

The method for purifying the target products, the aldehyde represented by Formula (1) and the aldehyde represented by Formula (2), from the solution after completion of the reaction is not particularly limited, and a known method may be appropriately selected and performed. Specifically, examples of the method include filtration, chromatography, and purification by distillation, and purification by appropriately combining these can provide a target aldehyde or aldehyde composition of high purity.

As described above, when the mass ratio between 4-isobutylbenzaldehyde and 2-isobutylbenzaldehyde [4-isobutylbenzaldehyde/2-isobutylbenzaldehyde] is from 97.00/3.00 to 99.999/0.001 as a product of the formylation step, it is preferable to use the product as a raw material as it is and set the mass ratio of positional isomers of the final product to the range (mass ratio between aldehyde represented by Formula (1) and aldehyde represented by Formula (2) [(1)/(2)]) to from 97.00/3.00 to 99.999/0.001, but the mass ratio may be adjusted to the range using a product after the hydrogenation step or a purified product obtained by producing the same as long as the mass ratio finally obtained is in the range.

### <Second Production Method>

A second production method is a method for producing an aldehyde composition including, in this order, a step of formylating isobutylbenzene with carbon monoxide under a superstrong acid condition to obtain 4-isobutylbenzaldehyde and 2-isobutylbenzaldehyde,
a step of acetalizing 4-isobutylbenzaldehyde and 2-isobutylbenzaldehyde in the presence of a para-toluenesulfonic acid catalyst,
a step of causing the resulting acetals to react with an alkyl vinyl ether under an acid catalyst,
a step of hydrolyzing in the presence of an acid catalyst to obtain an aldehyde represented by Formula (6) described below and an aldehyde represented by Formula (7) described below, and
a step of performing hydrogenation to obtain an aldehyde represented by Formula (1) described below and an aldehyde represented by Formula (2) described below.

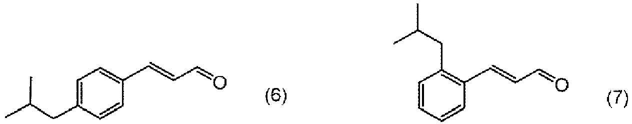

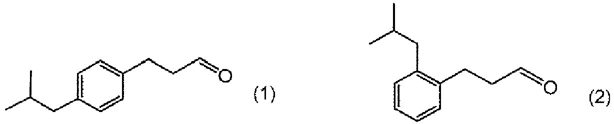

### (Formylation step)

The first step in the second production method is a step of formylating isobutylbenzene with carbon monoxide under a superstrong acid condition to obtain 4-isobutylbenzaldehyde and 2-isobutylbenzaldehyde. The formylation step is the same as the formylation step in the first production method, and preferable conditions are also the same. Hereinafter, a specific formylation step will be described. "Under a superstrong acid condition" means that a superstrong acid catalyst is used for the reaction.

The superstrong acid catalyst is preferably a combination of a Bronsted acid and a Lewis acid.

The Bronsted acid is not limited, but hydrogen fluoride, hydrogen chloride, and hydrogen bromide are preferable, and hydrogen fluoride is more preferable.

The Lewis acid is not limited, but boron trifluoride and aluminum chloride are preferable, and boron trifluoride is more preferable.

From the viewpoints of recovery and reuse of the catalyst and positional selectivity of the formyl group, a combination of hydrogen fluoride and boron trifluoride is even more preferable.

When hydrogen fluoride is used as the Bronsted acid, hydrogen fluoride also has a function as a solvent for the reaction. The hydrogen fluoride is preferably substantially anhydrous hydrogen fluoride from the perspective of the reactivity. The phrase "substantially anhydrous" means that the water content is 5 mass% or less, preferably 1 mass% or less, and more preferably 0.1 mass% or less.

The molar ratio of hydrogen fluoride to isobutylbenzene [hydrogen fluoride (mol)/isobutylbenzene (mol)] is preferably 3.0 or more, more preferably 5.0 or more, even more preferably 10.0 or more from the viewpoint of reactivity with carbon monoxide and suppression of side reactions, and is preferably 30.0 or less, more preferably 20.0 or less, even more preferably 15.0 or less from the viewpoint of economic efficiency and production efficiency.

When boron trifluoride is used as the Lewis acid, the molar ratio of boron trifluoride to isobutylbenzene [boron trifluoride (mol)/isobutylbenzene (mol)] is preferably 1.0 or more, more preferably 2.0 or more, and is preferably 4.0 or less, more preferably 3.0 or less, from the viewpoint of reactivity with carbon monoxide and suppression of side reactions.

The reaction temperature is preferably -50°C or more, more preferably -40°C or more, even more preferably -30°C or more, and is preferably 30°C or less, more preferably 0°C or less, even more preferably -10°C or less, from the viewpoint of improving the reactivity, suppressing side reactions, and improving the selectivity of the position at which the formyl group is introduced.

The reaction between isobutylbenzene and carbon monoxide is preferably performed under pressure. The pressure during the reaction is preferably 1.0 MPaG or more, more preferably 1.5 MPaG or more, and even more preferably 1.8 MPaG or more, and is preferably 3.0 MPaG or less, more preferably 2.5 MPaG or less, and even more preferably 2.2 MPaG or less, as a partial pressure of carbon monoxide, from the viewpoint of improving the reactivity and suppressing side reactions.

In the reaction (formylation reaction), the reaction time is not particularly limited, and is preferably 10 minutes or more, more preferably 20 minutes or more, and even more preferably 30 minutes or more, and is preferably 24 hours or less, more preferably 12 hours or less, and even more preferably 5 hours or less, from the viewpoint of sufficiently advancing the reaction, suppressing side reactions and decomposition of products, and efficient production.

The reaction may be performed in the presence of a solvent. The solvent to be used is not particularly limited as long as the solvent has good solubility of the reaction raw material and is inert to acid catalysts such as hydrogen fluoride and boron trifluoride. Examples thereof include saturated aliphatic hydrocarbons such as hexane, heptane, and decane, and halogenated aliphatic hydrocarbons such as chloroform, methylene chloride, and dichloroethane. One of these solvents may be used alone, or two or more of these may be used in combination. The amount of solvent to be used is not particularly limited, and is only required to be selected as appropriate from the perspective of the uniformity of the reaction, reaction rate, and solvent removal. When hydrogen fluoride is used in this step, the solvent does not have to be used because hydrogen fluoride also functions as a solvent.

The above reaction may be any method such as a batch type, a semi-batch type, a continuous type, and the like, but is preferably a continuous type, from the perspective that the catalyst can be recovered and recycled and the perspective of production efficiency.

The apparatus to be used in the production method is a reaction apparatus capable of sufficiently mixing the liquid phase and the gas phase while adjusting the temperature under pressure.

For example, in the continuous type, first, hydrogen fluoride and boron trifluoride are placed into a reactor equipped with a stirrer, the contents are stirred, the liquid temperature is set to a suitable temperature, and the temperature is kept constant. Then, the pressure is raised to a suitable reaction pressure with carbon monoxide, and carbon monoxide is allowed to be supplied so that the pressure is kept constant. Thereafter, a semi-batch type reaction in which isobutylbenzene dissolved in a solvent as necessary is supplied is performed. Further subsequently, supply of hydrogen fluoride, boron trifluoride, and isobutylbenzene dissolved in a solvent as necessary is started, and the reaction product solution is continuously extracted.

Hydrogen fluoride and boron trifluoride are removed from the obtained reaction product solution containing 4-isobutylbenzaldehyde and 2-isobutylbenzaldehyde, and as necessary, the reaction product solution is purified by distillation or extraction to obtain the target 4-isobutylbenzaldehyde and 2-isobutylbenzaldehyde.

When 2-isobutylbenzaldehyde is contained in the obtained 4-isobutylbenzaldehyde, further purification may be performed to obtain 4-isobutylbenzaldehyde of higher purity and 2-isobutylbenzaldehyde of higher purity, or a mixture thereof may be used as a raw material in the next step as it is.

In particular, when the mass ratio between 4-isobutylbenzaldehyde and 2-isobutylbenzaldehyde [4-isobutylbenzaldehyde/2-isobutylbenzaldehyde] is from 97.00/3.00 for 99.999/0.001, use of the product as it is as a raw material allows the mass ratio of the final product to be in the range (mass ratio between aldehyde represented by Formula (1) and aldehyde represented by Formula (2) [(1)/(2)]) of from 97.00/3.00 to 99.999/0.001, which is preferable.

The mass ratio between 4-isobutylbenzaldehyde and 2-isobutylbenzaldehyde [4-isobutylbenzaldehyde/2-isobutylbenzaldehyde] is preferably adjusted depending on the target scent. For example, when the purpose is to obtain the first aldehyde composition, the mass ratio is preferably from 97.00/3.00 to 99.90/0.10, more preferably from 98.00/2.00 to 99.90/0.10, even more preferably from 98.50/1.50 to 99.90/0.10, even more preferably from 99.00/1.00 to 99.80/0.20, and even more preferably from 99.20/0.80 to 99.70/0.30. The mass ratio is preferably from 97.00/3.00 to 99.999/0.001, more preferably from 98.00/2.00 to 99.999/0.001, even more preferably from 98.50/1.50 to 99.999/0.001, even more preferably from 99.50/0.50 to 99.998/0.002, even more preferably from 99.90/0.10 to 99.998/0.002, and even more preferably from 99.96/0.04 to 99.997/0.003, when it is intended to obtain the second aldehyde composition. The mass ratio is preferably from 97.00/3.00 to 99.999/0.001, more preferably from 98.00/2.00 to 99.999/0.001, even more preferably from 98.50/1.50 to 99.999/0.001, even more preferably from 99.00/1.00 to 99.995/0.005, even more preferably from 99.90/0.10 to 99.99/0.01, and even more preferably from 99.92/0.08 to 99.98/0.02, when it is intended to obtain the third aldehyde composition.

The mass ratio of positional isomers in the final product can be adjusted not only in this step but also in any step in the production method. Specifically, the ratio of positional isomers of the raw materials in each step corresponding to the target aldehyde may be adjusted.

### (Cinnamic aldehyde synthesis step using Muller-Conradi-Pieroh condition)

In the second production method, the next steps are a step of acetalizing 4-isobutylbenzaldehyde and 2-isobutylbenzaldehyde obtained in the previous step in the presence of a p-toluenesulfonic acid catalyst, a step of reacting the obtained acetals with an alkyl vinyl ether in the presence of an acid catalyst, and a step of hydrolyzing the acetals in the presence of an acid catalyst to obtain an aldehyde (isobutyl cinnamic aldehyde) represented by Formula (6) and an aldehyde (isobutyl cinnamic aldehyde) represented by Formula (7).

This step is a step of synthesizing isobutyl cinnamic aldehyde using Muller-Conradi-Pieroh conditions.

The acetalization step, which is the first step of this process, is specifically represented by the following formula:

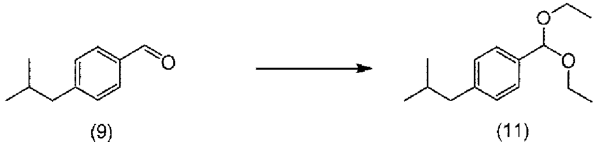

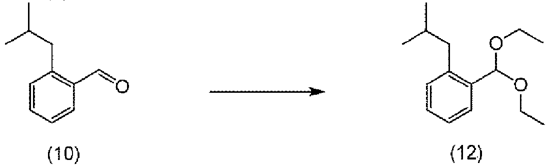

The 4-isobutylbenzaldehyde represented by Formula (9) and the 2-isobutylbenzaldehyde represented by Formula (10) are acetalized to obtain the acetal represented by Formula (11) and the acetal represented by Formula (12), respectively.

Acetalization can be performed by a known method. Examples thereof include a method of reacting methanol or ethanol under a p-toluenesulfonic acid catalyst and a method of reacting triethyl orthoformate under a p-toluenesulfonic acid catalyst.

Next, the method includes a step of reacting the obtained acetals with an alkyl vinyl ether in the presence of an acid catalyst to add the alkyl vinyl ether.

The alkyl vinyl ether addition step is specifically represented by the following formula: 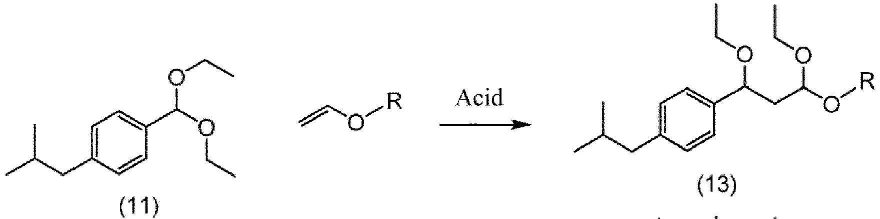 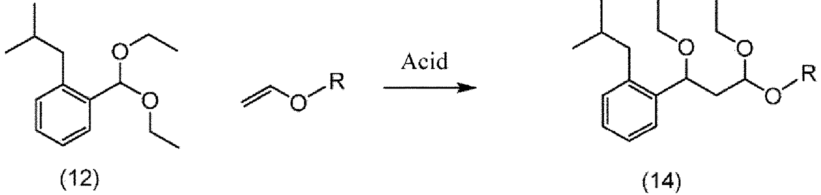 where in the formula, R represents an alkyl group having from 2 to 8 carbon atoms.

The acetal represented by Formula (11) and the acetal represented by Formula (12) are caused to react with an alkyl vinyl ether in the presence of an acid catalyst to add the alkyl vinyl ether, whereby the acetal represented by Formula (13) and the acetal represented by Formula (14) are respectively obtained.

The alkyl vinyl ether is preferably a vinyl ether having an alkyl group having from 2 to 8 carbon atoms, such as ethyl vinyl ether, propyl vinyl ether, isobutyl vinyl ether, cyclohexyl vinyl ether, or 2-ethylhexyl vinyl ether. Of these, from the viewpoint of reactivity, at least one selected from the group consisting of ethyl vinyl ether and propyl vinyl ether is more preferable.

Examples of the acid catalyst used in the alkyl vinyl ether addition step include, but are not limited to, Bronsted acids such as hydrochloric acid, hydrobromic acid, sulfuric acid, acetic acid and toluenesulfonic acid, and Lewis acids such as boron trifluoride, zinc chloride, and aluminum chloride.

The amount of the alkyl vinyl ether to be added is preferably 1 mol or more, more preferably 1.2 mol or more with respect to 1 mol of the substrate (acetal) from the viewpoint of reaction yield. From the viewpoint of economy, the amount is preferably 2 mol or less, more preferably 1.7 mol or less.

The reaction temperature is preferably 0°C or more, and more preferably 10°C or more from the viewpoint of reactivity. **In** addition, from the viewpoint of suppressing side reactions, the temperature is preferably 50°C or less, and more preferably 40°C or less.

Next, the method includes a step of hydrolyzing the acetals obtained in the alkyl vinyl ether addition step in the presence of an acid catalyst.

Specifically, the hydrolysis step is represented by the following formula: 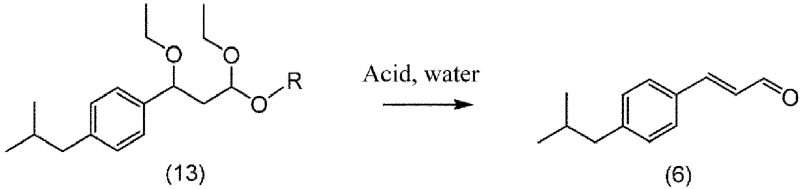 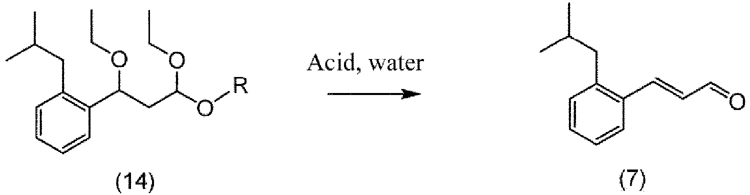 where in the formula, R represents an alkyl group having from 2 to 8 carbon atoms.

The acetal represented by Formula (13) and the acetal represented by Formula (14) are hydrolyzed under the presence of an acid catalyst to obtain the aldehyde represented by Formula (6) and the aldehyde represented by Formula (7), respectively.

Examples of the acid catalyst used in this hydrolysis step include, but are not limited to, hydrochloric acid, hydrobromic acid, sulfuric acid, acetic acid, and toluenesulfonic acid.

The amount of the acid catalyst to be added is preferably 0.5 mass% or more, more preferably 1 mass% or more, and even more preferably 3 mass% or more with respect to the substrate (acetal) from the viewpoint of efficiency of the hydrolysis reaction. Further, from the viewpoint of suppressing side reactions, the amount is preferably 20 mass% or less, more preferably 15 mass% or less.

The reaction temperature is preferably 60°C or more, more preferably 80°C or more. Further, the reaction is preferably performed under reflux. The reaction temperature is preferably 100°C or less.

### (Hydrogenation step)

In the second production method, the next step is a step of performing hydrogenation to obtain the aldehyde represented by Formula (1) and the aldehyde represented by Formula (2).

This hydrogenation step is a step of hydrogenating the aldehyde represented by Formula (6) and the aldehyde represented by Formula (7) (isobutyl cinnamic aldehyde) obtained in the previous step to obtain a target aldehyde composition containing the aldehyde represented by Formula (1) and the aldehyde represented by Formula (2). The present hydrogenation step is the same as the hydrogenation step in the first production method, and preferable conditions are also the same. A specific hydrogenation step will be described below.

The hydrogenation method is not particularly limited but can be performed by a known method using a hydrogenation catalyst.

The hydrogenation catalyst is not particularly limited, and a known catalyst can be used. The hydrogenation catalyst is not particularly limited, and a known catalyst can be used, including, for example, a supported heterogeneous hydrogenation catalyst in which a metal, such as Ni, Pt, Pd, or Ru, is supported on carbon, silica, alumina, diatomaceous earth, or the like; what is called a Ziegler-type hydrogenation catalyst prepared using a transition metal salt, such as an organic acid salt and an acetylacetone salt of Ni, Co, Fe, Cr, or the like, , and a reducing agent, such as organoaluminum; and a homogeneous hydrogenation catalyst of what is called an organometallic complex or the like, such as an organometallic compound of Ti, Ru, Rh, Zr, or the like.

The temperature of the hydrogenation reaction in this step is preferably 0°C or more, more preferably 10°C or more, and even more preferably 20°C or more, and is preferably 150°C or less and more preferably 100°C or less from the viewpoints of the reactivity and suppressing side reactions.

The pressure of hydrogen used in the hydrogenation reaction is preferably 0.01 MPaG or more, more preferably 0.03 MPaG or more, and even more preferably 0.05 MPaG or more, and is preferably 10 MPaG or less, more preferably 3 MPaG or less, even more preferably 1 MPaG or less, and still more preferably 0.5 MPaG or less.

The reaction time is not particularly limited but is preferably 3 minutes or more, more preferably 10 minutes or more, and even more preferably 30 minutes or more, and is preferably 24 hours or less, more preferably 12 hours or less, and even more preferably 8 hours or less.

The hydrogenation reaction may be performed in the presence of a solvent. The solvent to be used is not particularly limited as long as the hydrogenation reaction is not inhibited but is exemplified by hydrocarbon solvents including: aliphatic hydrocarbons, such as pentane, hexane, isopentane, heptane, octane, and isooctane; aliphatic hydrocarbons, such as cyclopentane, methylcyclopentane, cyclohexane, methylcyclohexane, and ethylcyclohexane; and aromatic hydrocarbons, such as benzene, toluene, ethylbenzene, and xylene. One of these may be used individually, or two or more of these may be used in combination.

The method for purifying the target products, the aldehyde represented by Formula (1) and the aldehyde represented by Formula (2), from the solution after completion of the reaction is not particularly limited, and a known method may be appropriately selected and performed. Specifically, examples of the method include filtration, chromatography, and purification by distillation, and purification by appropriately combining these can provide a target aldehyde or aldehyde composition of high purity.

As described above, when the mass ratio between 4-isobutylbenzaldehyde and 2-isobutylbenzaldehyde [4-isobutylbenzaldehyde/2-isobutylbenzaldehyde] is from 97.00/3.00 to 99.999/0.001 as a product of the formylation step, it is preferable to use the product as a raw material as it is and set the mass ratio of positional isomers of the final product to the range (mass ratio between aldehyde represented by Formula (1) and aldehyde represented by Formula (2) [(1)/(2)]) to from 97.00/3.00 to 99.999/0.001, but the mass ratio may be adjusted to the range using a product after the hydrogenation step or a purified product obtained by producing the same as long as the mass ratio finally obtained is in the range.

### Examples

The present invention will be described in detail based on Examples described below, but the present invention is not limited to these Examples.

### [Analysis and Evaluation]

### <Gas chromatography analysis (analysis of constitution)>

The constitution in each step described later, the constitution of the product, and the constitution of the composition were determined using gas chromatograph (GC-2010 Plus, available from Shimadzu Corporation) by preparing a calibration curve using n-decane (reagent grade, available from FUJIFILM Wako Pure Chemical Corporation) as an internal standard material.

A capillary column HR-1701 (0.32 mm φ in inner diameter and 30 m in length) available from Agilent Technologies Japan, Ltd. was used. The heating program: increasing the temperature from 100°C to 280°C at a rate of 5°C/min and maintaining for 30 minutes

### <Evaluation of scent/aromatic note>

The scent and aromatic note of aldehyde compositions and fragrance compositions obtained in examples and comparative examples were evaluated by a method in which filter paper of 8 mm in width and 15 cm in length was impregnated with the composition, and an expert panelist sniffed the filter paper.

Among the aromatic notes, with respect to floral feeling, based on Example 1, those equivalent to Example 1 were rated as A, those stronger than Example 1 were rated as A+, those weaker than Example 1 were rated as B, and those having no floral feeling were rated as C.

Among the aromatic notes, with respect to the aldehyde feeling, based on Example 1, those equivalent to Example 1 were rated as A, those stronger than Example 1 were rated as A+, those weaker than Example 1 were rated as B, and those having no aldehyde feeling were rated as C.

Among the aromatic notes, with respect to the powdery feeling, those having the powdery feeling were given P.

### <Evaluation of threshold of smell>

Each of the aldehyde compositions obtained in the examples and Lilial (3-(p-tert-butylphenyl)-2-methylpropanal) were diluted with dipropylene glycol to have concentrations of 10 ppm, 1 ppm, 0.1 ppm, 0.01 ppm and 0.001 ppm, immersed in filter paper having a width of 8 mm and a length of 15 cm, and a lower limit concentration at which the smell can be identified by a method in which a specialized panelist sniffs was evaluated. As the lower limit concentration is lower, the threshold of smell is lower, and the intensity and diffusibility of the aroma are more excellent.

### <Evaluation of lasting property>

Each of the aldehyde compositions obtained in Examples and Lilial (3-(p-tert-butylphenyl)-2-methylpropanal) was impregnated into a filter paper having a width of 8 mm and a length of 15 cm, and the longest period during which smell was able to be detected by a method in which a specialized panelist sniffs was evaluated every week. The longer the longest period during which smell can be detected, the better the lasting property.

### <Carcinogenicity prediction test>

Using the aldehyde compositions obtained in Examples and Lilial (3-(p-tert-butylphenyl)-2-methylpropanal), a transformation test (an in vitro carcinogenicity prediction test using Bhas42 cells) was performed based on OECD 231GD to determine whether the carcinogenicity was negative or positive. When the result of this test is negative, it is predicted that there is no possibility of causing carcinogenicity, and safety is high.

### [Synthesis of raw material]

### Production Example 1 (Synthesis of isobutylbenzaldehyde)

The following synthesis was performed using a stainless steel autoclave having an internal volume of 10 L equipped with a magnetic induction type stirrer, three inlet nozzles at the top, and one extraction nozzle at the bottom, the internal temperature of which can be controlled by a jacket. First, the inside of the autoclave was purged with carbon monoxide, then hydrogen fluoride (available from Morita Chemical Industry Co., Ltd., 1128 g, 56.4 mol) and boron trifluoride (available from STELLACHEMIFA CORPORATION, 765 g, 11.3 mol) were added, the liquid temperature was set to -25°C, and then the pressure was increased to 2 MPa with carbon monoxide. Isobutylbenzene (available from Tokyo Chemical Industry Co., Ltd., 946 g, 7.05 mol) was supplied from the upper part of the autoclave over 60 minutes while maintaining the reaction temperature at -25°C and the reactor pressure at 2 MPa, and stirring was continued for about 30 minutes until absorption of carbon monoxide was no longer observed. The reacted mixture in the autoclave was discharged into ice water. The extracted mixture was shaken well, and then the oil layer was separated. The obtained oil layer part was washed with water, and then the obtained oil layer part was purified by distillation using a rectification column with a theoretical plate number of 20 (95°C, 3 Torr) to obtain isobutylbenzaldehyde (purity: 99.5 mass%, mass ratio between 4-isobutylbenzaldehyde and 2-isobutylbenzaldehyde [(4-isobutylbenzaldehyde)/(2-isobutylbenzaldehyde)] = 99.95/0.05, 650 g) as a colorless transparent liquid.

### Production Example 2 (Synthesis of isobutylbenzaldehyde)

The following synthesis was performed using a stainless steel autoclave having an internal volume of 500 mL equipped with a magnetic induction type stirrer, three inlet nozzles at the top, and one extraction nozzle at the bottom, the internal temperature of which can be controlled by a jacket. First, isobutylbenzene (100 g, 0.75 mol, available from Tokyo Chemical Industry Co., Ltd.), 1,2-dichloroethane (295 g, available from FUJIFILM Wako Pure Chemical Corporation), aluminum chloride (149 g, available from FUJIFILM Wako Pure Chemical Corporation), and 37% hydrochloric acid (1.0 g, available from FUJIFILM Wako Pure Chemical Corporation) were charged, and the inside of the autoclave was replaced with carbon monoxide. The liquid temperature was adjusted to 20°C, and then the pressure was increased to 2 MPa with carbon monoxide. Stirring was continued for about 60 minutes until no absorption of carbon monoxide was observed while maintaining the reaction temperature at 20°C and the reaction pressure at 2 MPa. The reacted mixture in the autoclave was discharged into ice water. The extracted mixture was shaken well, and then the oil layer was separated. The obtained oil layer part was washed with water, and then the obtained oil layer part was purified by distillation using a rectification column with a theoretical plate number of 20 (95°C, 3 Torr) to obtain isobutylbenzaldehyde (purity: 99.5 mass%, mass ratio between 4-isobutylbenzaldehyde and 2-isobutylbenzaldehyde [(4-isobutylbenzaldehyde)/(2-isobutylbenzaldehyde)] = 99.0/1.0, 50 g) as a colorless transparent liquid.

### Production Example 3 (Synthesis of isobutylbenzaldehyde)

The following synthesis was performed using a stainless steel autoclave having an internal volume of 10 L equipped with a magnetic induction type stirrer, three inlet nozzles at the top, and one extraction nozzle at the bottom, the internal temperature of which can be controlled by a jacket. First, isobutylbenzene (1000 g, 7.5 mol, available from Tokyo Chemical Industry Co., Ltd.), 1,2-dichloroethane (2950 g, available from FUJIFILM Wako Pure Chemical Corporation), aluminum chloride (1490 g, available from FUJIFILM Wako Pure Chemical Corporation), and 37% hydrochloric acid (10 g, available from FUJIFILM Wako Pure Chemical Corporation) were charged, and the inside of the autoclave was replaced with carbon monoxide. The liquid temperature was adjusted to 20°C, and then the pressure was increased to 2 MPa with carbon monoxide. Stirring was continued for about 60 minutes until no absorption of carbon monoxide was observed while maintaining the reaction temperature at 20°C and the reaction pressure at 2 MPa. The reacted mixture in the autoclave was discharged into ice water. The extracted mixture was shaken well, and then the oil layer was separated. The obtained oil layer part was washed with water, and then the obtained oil layer part was purified by distillation using a rectification column with a theoretical plate number of 20 (from 96 to 97°C, 3 Torr) to obtain isobutylbenzaldehyde (purity: 98.5 mass%, mass ratio between 4-isobutylbenzaldehyde and 2-isobutylbenzaldehyde [(4-isobutylbenzaldehyde)/(2-isobutylbenzaldehyde)] = 95.5/4.5, 205 g) as a colorless transparent liquid.

### [Production of aldehyde composition]

### Example 1 (production of aldehyde composition containing 3-(4-isobutylphenyl)propanal and 3-(2-isobutylphenyl)propanal: method using aldol condensation)

### (Aldol condensation step)

Methanol (400.0 g), sodium hydroxide (available from FUJIFILM Wako Pure Chemical Corporation, 18.1 g), and isobutylbenzaldehyde (200.0 g) obtained in Production Example 1 were charged into a round-bottom flask having an internal volume of 1000 mL equipped with a stirrer, a thermometer, and a dropping funnel, and cooled to 10°C with stirring, and then acetaldehyde (available from FUJIFILM Wako Pure Chemical Corporation, 56.4 g) was added dropwise thereto over 3 hours. After completion of the dropwise addition, the mixture was maintained at 10°C for 1 hour, and the reaction was completed. After neutralization by adding acetic acid (16.4 g), water and heptane were added, the mixture was shaken, and the aqueous phase was separated and removed by liquid separation. Heptane was then distilled off, and a crude intermediate was obtained. This crude intermediate was subjected to simple distillation (from 154 to 161°C/7 torr) to obtain 3-(isobutylphenyl)propenal (54.0 g, purity 94.0 mass%).

### (Hydrogenation step)

The 3-(isobutylphenyl)propenal (50.0 g) obtained in the aldol condensation step, 2-propanol (30.0 g), and a 5% palladium-carbon catalyst (available from N. E. CHEMCAT CORPORATION, water-containing product, PE type, 0.5 g) were charged into a 200 mL stainless steel autoclave equipped with a magnetic induction stirrer and capable of controlling the internal temperature by a jacket, and stirred at 25°C and a hydrogen pressure of 0.05 MPa for 5 hours to perform a hydrogenation reaction. The reaction solution was filtered to remove the catalyst, heptane was added, the mixture was shaken, and the aqueous phase was separated and removed by liquid separation. Heptane was then distilled off, and a crude composition was obtained. The crude composition was rectified at 1 torr using a rectification column with 20 theoretical plates to obtain an aldehyde composition (8.0 g, mass ratio [3-(4-isobutylphenyl)propanal/3-(2-isobutylphenyl)propanal] = 99.95/0.05) containing 3-(4-isobutylphenyl)propanal and 3-(2-isobutylphenyl)propanal as a fraction at 108 to 110°C. The evaluation results of the scent/aromatic note of the resulting aldehyde compositions are presented in Table 1.

### Example 2 (production of aldehyde composition containing 3-(4-isobutylphenyl)propanal and 3-(2-isobutylphenyl)propanal: method using Muller-Conradi-Pieroh condition)

### (Step of adding ethyl vinyl ether to acetal under Muller-Conradi-Pieroh condition)

Triethyl orthoformic acid (87.2 g, available from FUJIFILM Wako Pure Chemical Corporation) and the isobutylbenzaldehyde (86.0 g) obtained in Production Example 1 were charged into a round-bottom flask having an internal volume of 300 mL equipped with a stirrer, a thermometer, and a dropping funnel, and cooled to 10°C with stirring, then p-toluenesulfonic acid monohydrate (0.5 g, available from FUJIFILM Wako Pure Chemical Corporation) was added thereto, and the temperature was raised to 25°C. The obtained material was held at 25°C for 30 minutes. Thereafter, a boron trifluoride diethyl ether complex (available from FUJIFILM Wako Pure Chemical Corporation, 0.2 g) was added thereto, and ethyl vinyl ether (available from Tokyo Chemical Industry Co., Ltd., 57.3 g) was added dropwise thereto over 4 hours. After completion of the dropwise addition, the mixture was maintained for 1 hour, and the reaction was completed. Sodium acetate (4.0 g) was added for neutralization, and then low boiling components were distilled off. To the obtained crude reaction solution, 37% HCl (available from FUJIFILM Wako Pure Chemical Corporation, 8.0 g) and water (50.0 g) were added, and the mixture was heated to 90°C and stirred for 24 hours. Thereafter, heptane was added, and the aqueous phase was separated by liquid separation. Heptane was then distilled off, and a crude intermediate was obtained. This crude intermediate was subjected to simple distillation (from 156 to 161°C/7 torr) to obtain 3-(isobutylphenyl)propenal (54.0 g, purity 95.4 mass%).

### (Hydrogenation step)

The 3-(isobutylphenyl)propenal (50.0 g) obtained in the previous step, 2-propanol (30.0 g), and a 5% palladium-carbon catalyst (available from N. E. CHEMCAT CORPORATION, water-containing product, PE type, 0.5 g) were charged into a 200 mL stainless steel autoclave equipped with a magnetic induction stirrer and capable of controlling the internal temperature by a jacket, and stirred at 25°C and a hydrogen pressure of 0.05 MPa for 5 hours to perform a hydrogenation reaction. The reaction solution was filtered to remove the catalyst, heptane was added, the mixture was shaken, and the aqueous phase was separated and removed by liquid separation. Heptane was then distilled off, and a crude composition was obtained. The crude composition was rectified at 1 torr using a rectification column with 20 theoretical plates to obtain an aldehyde composition (9.3 g) containing 3-(4-isobutylphenyl)propanal and 3-(2-isobutylphenyl)propanal as fractions at from 108 to 110°C. The evaluation results of the scent/aromatic note of the resulting aldehyde compositions are presented in Table 1.

### Example 3 (production of aldehyde composition containing 3-(4-isobutylphenyl)propanal and 3-(2-isobutylphenyl)propanal: method using Muller-Conradi-Pieroh condition)

### (Step of adding ethyl vinyl ether to acetal under Muller-Conradi-Pieroh condition)

Obtained was 3-(isobutylphenyl)propenal (52.0 g, purity 94.4 mass%) in the same manner as in Example 2 except that 3.5 g of p-toluenesulfonic acid monohydrate was used, and after reacting with triethylorthoformic acid, a boron trifluoride diethyl ether complex was not added.

### (Hydrogenation step)

Using 3-(isobutylphenyl)propenal (50.0 g) obtained in the previous step, an aldehyde composition (9.0 g) containing 3-(4-isobutylphenyl)propanal and 3-(2-isobutylphenyl)propanal was obtained in the same manner as in Example 2. The evaluation results of the scent/aromatic note of the resulting aldehyde compositions are presented in Table 1.

### Example 4 (production of aldehyde composition containing 3-(4-isobutylphenyl)propanal and 3-(2-isobutylphenyl)propanal)

An aldehyde composition (0.9 g) containing 3-(4-isobutylphenyl)propanal and 3-(2-isobutylphenyl)propanal was obtained by increasing the content of 3-(4-isobutylphenyl)propanal in the composition obtained in Example 1, using column chromatography (mass ratio [3-(4-isobutylphenyl) propanal/3-(2-isobutylphenyl) propanal]= 99.996/0.004). Scent and aromatic note were evaluated. The results are presented in Table 1.

### Example 5 (production of aldehyde composition containing 3-(4-isobutylphenyl)propanal and 3-(2-isobutylphenyl)propanal: method using Muller-Conradi-Pieroh condition)

### (Step of adding ethyl vinyl ether to acetal under Muller-Conradi-Pieroh condition)

An aldehyde composition (8.0 g) containing 3-(4-isobutylphenyl)propanal and 3-(2-isobutylphenyl)propanal was obtained in the same manner as in Example 2 except that the isobutylbenzaldehyde (50.0 g) obtained in Production Example 2 was used. The evaluation results of the scent/aromatic note of the resulting aldehyde compositions are presented in Table 1.

### Example 6

The aldehyde composition obtained in Example 2 and the aldehyde composition obtained in Example 5 were mixed at a mass ratio of 1:1 to obtain an aldehyde composition containing 3-(4-isobutylphenyl)propanal and 3-(2-isobutylphenyl)propanal. The evaluation results of the scent/aromatic note of the resulting aldehyde compositions are presented in Table 1.

### Example 7 (production of aldehyde composition containing 3-(4-isobutylphenyl)propanal and 3-(2-isobutylphenyl)propanal: method using Muller-Conradi-Pieroh condition)

### (Step of adding ethyl vinyl ether to acetal under Muller-Conradi-Pieroh condition)

A crude reaction solution was obtained in the same manner as in Example 2 using isobutylbenzaldehyde (50.0 g) obtained in Production Example 1. The obtained crude reaction solution was used as it was in the hydrogenation step.

### (Hydrogenation step)

Using the crude reaction solution (50.0 g) obtained in the previous step, an aldehyde composition (4.0 g) containing 3-(4-isobutylphenyl)propanal and 3-(2-isobutylphenyl)propanal was obtained in the same manner as in Example 2. The evaluation results of the scent/aromatic note of the resulting aldehyde compositions are presented in Table 1.

### Example 8

An aldehyde composition (0.8 g) containing 3-(4-isobutylphenyl)propanal and 3-(2-isobutylphenyl)propanal was obtained by increasing the content of the ester compound (Formula (4)) in the composition obtained in Example 2 using column chromatography. The evaluation results of the scent/aromatic note of the resulting aldehyde compositions are presented in Table 1.

### Example 9

An aldehyde composition was prepared by a method of adding ethyl paratoluenesulfonate (available from FUJIFILM Wako Pure Chemical Corporation) to the aldehyde composition obtained in Example 3. The evaluation results of the scent/aromatic note of the resulting aldehyde compositions are presented in Table 1.

### Example 10

The aldehyde composition obtained in Example 1 and the aldehyde composition obtained in Comparative Example 1 were mixed at a mass ratio of 6:4 to obtain an aldehyde composition containing 3-(4-isobutylphenyl)propanal and 3-(2-isobutylphenyl)propanal. The evaluation results of the scent/aromatic note of the resulting aldehyde compositions are presented in Table 1.

### Comparative Example 1

An aldehyde composition (7.0 g) containing 3-(4-isobutylphenyl)propanal and 3-(2-isobutylphenyl)propanal was obtained in the same manner as in Production Example 1 except that the isobutylbenzaldehyde (200.0 g) obtained in Production Example 3 was used. The evaluation results of the scent/aromatic note of the resulting aldehyde compositions are presented in Table 1.

**[Table 1-1]**

| Table 1 | | | | | | |
|---|---|---|---|---|---|---|
| | Example 1 | Example 2 | Example 3 | Example 4 | Example 5 | Example 6 |
| Embodiment of aldehyde composition | Second aldehyde composition | Third aldehyde composition | - | Second aldehyde composition | First aldehyde composition | First aldehyde composition |
| 3-(4-isobutylphenyl) propanal (Formula (1)) (mass%) | 98.42 | 97.40 | 97.30 | 99.32 | 95.79 | 96.57 |
| 3-(2-isobutylphenyl) propanal (Formula (2)) (mass%) | 0.05 | 0.05 | 0.05 | 0.004 | 0.93 | 0.47 |
| Alcohol compound (Formula (3)) (mass%) | 0.30 | 0.26 | 0.48 | 0.00 | 0.18 | 0.21 |
| Ester compound (Formula (4)) (mass%) | 0.00 | 0.50 | 0.20 | 0.02 | 0.41 | 0.37 |
| Sulfonic acid ester compound (Formula (5)) (mass%) | 0.00 | 0.02 | 0.51 | 0.00 | 0.00 | 0.00 |
| (1)/(2) (Mass ratio) | 99.95/0.05 | 99.95/0.05 | 99.95/0.05 | 99.996/0.004 | 99.04/0.96 | 99.52/0.48 |
| Others | 1.23 | 1.77 | 1.46 | 0.66 | 2.70 | 2.38 |
| Scent/aromatic note | Green, Aldehydic, Floral, Aquatic, Hyacinth | Green, Aldehydic, Floral, Leafy, Powdery | Green, Aldehydic, Floral, Aquatic, Hyacinth | Green, Aldehydic, Floral, Aquatic, Hyacinth | Green, Floral, Aldehyde Leafy, Aquatic | Green, Floral, Aldehyde Leafy, Aquatic |
| Floral feeling | A | B | A | A | A+ | A+ |
| Aldehyde feeling | A | A | B | A | B | B |
| Powdery feeling | - | P | - | - | - | - |
| Comparison with Example 1 | | It has a dry green feeling and a slightly weak floral feeling. | It has a hyacinth green and floral feeling and a slightly weak fatty aldehyde feeling. | Equivalent | The fatty aldehyde feeling is weak, and the floral feeling is strong. | The green and fatty aldehyde feeling is weak, and the floral feeling is strong. |

**[Table 1-2]**

| Table 1 | | | | | |
|---|---|---|---|---|---|
| | Example 7 | Example 8 | Example 9 | Example 10 | Comparative Example 1 |
| Embodiment of aldehyde composition | - | Third aldehyde composition | - | Second aldehyde composition | - |
| 3-(4-isobutylphenyl) propanal (Formula (1)) (mass%) | 96.81 | 96.35 | 96.79 | 96.22 | 92.87 |
| 3-(2-isobutylphenyl) propanal (Formula (2)) (mass%) | 0.05 | 0.05 | 0.05 | 1.76 | 4.07 |
| Alcohol compound (Formula (3)) (mass%) | 0.99 | 0.23 | 0.48 | 0.20 | 0.08 |
| Ester compound (Formula (4)) (mass%) | 0.20 | 1.00 | 0.20 | 0.00 | 0.00 |
| Sulfonic acid ester compound (Formula (5)) (mass%) | 0.09 | 0.02 | 1.06 | 0.00 | 0.00 |
| (1)/(2) (Mass ratio) | 99.95/0.05 | 99.95/0.05 | 99.95/0.05 | 98.20/1.80 | 95.80/4.20 |
| Others | 1.86 | 2.35 | 1.42 | 1.82 | 2.98 |
| Scent/aromatic note | Green, Aldehydic, Floral, Leafy, Aquatic | Green, Leafy, Floral, Aldehyde, Powdery | Green, Aldehydic, Floral, Hyacinth, Aquatic | Green, Floral, Aldehyde Leafy, Aquatic | Green, Floral, Leafy, Oily, Metalic |
| Floral feeling | B | B | A | B | B |
| Aldehyde feeling | B | B | B | A | C |
| Powdery feeling | - | P | - | - | - |
| Comparison with Example 1 | The green feeling is strong, and the floral, fatty aldehyde, and aquatic feelings are weak. | The green feeling is strong, and the floral, fatty aldehyde, and aquatic feelings are weak. | The green feeling is strong, and the fatty aldehyde and aquatic feeling are weak. | The green feeling is strong and the floral feeling is slightly weak. | Metalic green feeling is strong, floral feeling is weak, and there is no aldehyde or aquatic feeling. |

The aldehyde compositions of the examples had a scent with a good balance of green, aldehyde, and floral feeling. Further, the first aldehyde composition (Examples 5 and 6) had a scent excellent in balance of green, aldehyde, and floral, and also had a stronger floral feeling. The second aldehyde composition (Examples 1, 4 and 10) had a stronger aldehyde feeling while having a scent excellent in balance of green, aldehyde, and floral. The third aldehyde composition (Examples 2 and 8) had a scent excellent in balance of green, aldehyde, and floral, and further had a powdery feeling. It can be seen that the aldehyde composition of the examples is useful as a fragrance because it has an excellent scent, as described above.

### [Evaluation of threshold of smell, evaluation of lasting property, and carcinogenicity prediction test]

The aldehyde composition of Example 1 was subjected to evaluation of threshold of smell, evaluation of lasting property, and carcinogenicity prediction test. As Comparative Example 2, Lilial (3-(p-tert-butylphenyl)-2-methylpropanal) was subjected to evaluation of the threshold of smell, evaluation of lasting property, and carcinogenicity prediction test.

### [Table 2]

**Table 2**

| | | Threshold of smell (ppm) | Lasting property | Carcinogenicity prediction test |
|---|---|---|---|---|
| Example 1 | Aldehyde composition ((1)/(2) = 99.95/0.05) | 0.01 | 4 weeks | Negative |
| Comparative Example 2 | Lilial (3-(p-tert-butylphenyl)-2-methylpropanal) | 1 | 3 weeks | Positive |

It is found that the aldehyde compositions of Examples have a lower threshold of smell than Lilial having a similar structure and aromatic note, and are superior in intensity and diffusibility of the aroma. In addition, it is found that the aldehyde compositions of Examples are superior to Lilial in terms of lasting property. Further, the aldehyde compositions of Examples were negative in the carcinogenicity prediction test, indicating that they are also excellent in safety.

### Example 11 and Comparative Example 3 (lemonglass-like fragrance composition)

As Example 11, the aldehyde composition of Example 1 was added to the compounded fragrance base presented in Table 3 so as to be 1 mass%, and the scent/aromatic note were evaluated. As Comparative Example 3, MEFRANAL (3-methyl-5-phenyl-1-pentanal, floral scent with aldehyde and green feeling) was added in an amount of 1 mass% to the compounded fragrance base described in Table 3 to evaluate scent/aromatic note.

**[Table 3]**

| Table 3 | | (Parts by mass) |
|---|---|---|
| | Example 11 | Comparative Example 3 |
| MEFRANAL (3-METHYL-5-PHENYL-1-PENTANAL) | - | 10 |
| ALDEHYDE COMPOSITION OF EXAMPLE 1 ((1)/(2) = 99.95/0.05) | 10 | - |
| ALDEHYDE C-10 | 6 | 6 |
| BLACK PEPPER OIL INDIA | 0.8 | 0.8 |
| CARYOPHYLLENE | 40 | 40 |
| CITRAL SYNTHETIC | 640 | 640 |
| CITRONELLA OIL | 50 | 50 |
| CITRONELLAL | 8 | 8 |
| CITRONELLOL | 8 | 8 |
| CITRONELLYL NITRILE | 2 | 2 |
| CUMINIC ALDEHYDE | 0.8 | 0.8 |
| DPG | 98 | 98 |
| GERANIOL | 40 | 40 |
| GERANYL ACETATE | 10 | 10 |
| GINGER OIL CHINESE | 2 | 2 |
| ISO EUGENOL | 4 | 4 |
| LEMONILE | 2 | 2 |
| LIME TERPENE | 10 | 10 |
| LINALOOL | 6 | 6 |
| METHYL HEPTENONE | 10 | 10 |
| ORANGE OIL FLORIDA | 40 | 40 |
| PARA CYMENE | 4 | 4 |
| PLICATONE 974482 | 2 | 2 |
| TAGETES OIL | 0.4 | 0.4 |
| TERPINOLENE 20 | 6 | 6 |
| | 1000 | 1000 |

As a result of the evaluation of scent/aromatic note, the fragrance composition of Example 11 had a softer natural feeling than MEFRANAL having a hard impression of aromatic note, and had an effect of adding a gentle floral feeling as a whole. In addition, the diffusibility greatly exceeded that of the fragrance composition of Comparative Example 3.

From the results of Table 3, it is found that the fragrance composition of the present invention containing the aldehyde composition has increased the scent described above and is further excellent in diffusibility. That is, it is found that the aldehyde composition of the present invention can impart, in addition to the scent described above, diffusibility to a fragrance composition.

## Claims

1. An aldehyde composition, comprising an aldehyde represented by Formula (1) described below and an aldehyde represented by Formula (2) described below, wherein a mass ratio [(1)/(2)] between the aldehyde represented by Formula (1) and the aldehyde represented by Formula (2) is from 97.00/3.00 to 99.999/0.001

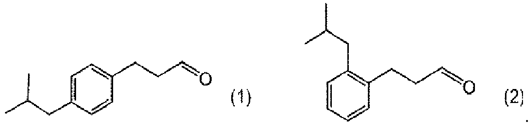

2. The aldehyde composition according to claim 1, wherein the composition comprises the aldehyde represented by Formula (1) described below and the aldehyde represented by Formula (2) described below, and the mass ratio [(1)/(2)] between the aldehyde represented by Formula (1) and the aldehyde represented by Formula (2) is from 97.00/3.00 to 99.90/0.10

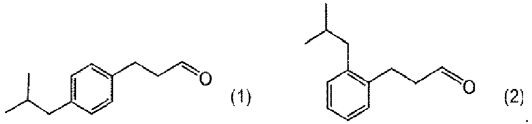

3. The aldehyde composition according to claim 1, wherein the composition comprises the aldehyde represented by Formula (1) described below and the aldehyde represented by Formula (2) described below, the mass ratio [(1)/(2)] between the aldehyde represented by Formula (1) and the aldehyde represented by Formula (2) is from 97.00/3.00 to 99.999/0.001, and a content of an ester represented by Formula (4) described below is 0.10 mass% or less with respect to a total amount of the aldehyde represented by Formula (1) and the aldehyde represented by Formula (2)

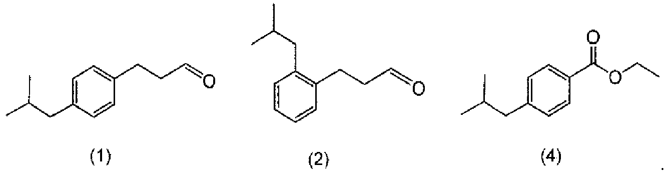

4. The aldehyde composition according to claim 1, wherein the composition comprises the aldehyde represented by Formula (1) described below, the aldehyde represented by Formula (2) described below, an ester represented by Formula (4) described below, the mass ratio [(1)/(2)] between the aldehyde represented by Formula (1) and the aldehyde represented by Formula (2) is from 97.00/3.00 to 99.999/0.001, and a content of the ester represented by Formula (4) is from 0.50 to 2.00 mass% or less with respect to a total amount of the aldehyde represented by Formula (1) and the aldehyde represented by Formula (2)

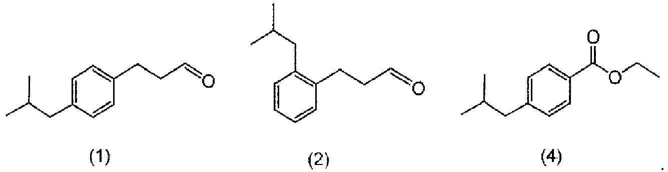

5. The aldehyde composition according to claim 1, wherein a total purity of the aldehydes represented by Formula (1) and Formula (2) is 97 mass% or more.

6. The aldehyde composition according to claim 1, wherein the composition comprises an alcohol represented by Formula (3) described below in an amount from 0.01 to 0.90 mass%

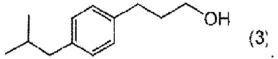

7. The aldehyde composition according to claim 1, wherein the composition comprises a sulfonic acid ester represented by Formula (5) described below in an amount from 0.01 to 0.90 mass%

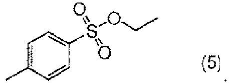

8. A fragrance composition comprising the aldehyde composition described in any one of claims 1 to 7.

9. A method for producing the aldehyde composition described in claim 1, the method comprising, in this order:
a step of formylating isobutylbenzene with carbon monoxide under a superstrong acid condition to obtain 4-isobutylbenzaldehyde and 2-isobutylbenzaldehyde;
a step of performing aldol condensation of 4-isobutylbenzaldehyde and 2-isobutylbenzaldehyde with acetaldehyde to obtain an aldehyde represented by Formula (6) described below and an aldehyde represented by Formula (7) described below; and
a step of performing hydrogenation to obtain an aldehyde represented by Formula (1) described below and an aldehyde represented by Formula (2) described below

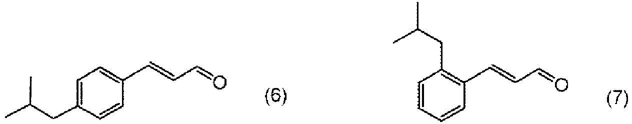

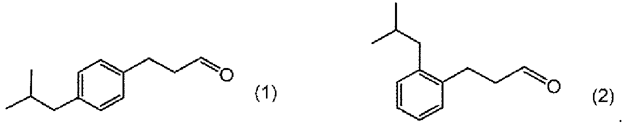

10. A method for producing the aldehyde composition described in claim 1, the method comprising, in this order:
a step of formylating isobutylbenzene with carbon monoxide under a superstrong acid condition to obtain 4-isobutylbenzaldehyde and 2-isobutylbenzaldehyde;
a step of acetalizing 4-isobutylbenzaldehyde and 2-isobutylbenzaldehyde in the presence of a para-toluenesulfonic acid catalyst;
a step of causing acetals obtained to react with an alkyl vinyl ether under an acid catalyst;
a step of hydrolyzing in the presence of an acid catalyst to obtain an aldehyde represented by Formula (6) described below and an aldehyde represented by Formula (7) described below; and
a step of performing hydrogenation to obtain an aldehyde represented by Formula (1) described below and an aldehyde represented by Formula (2) described below

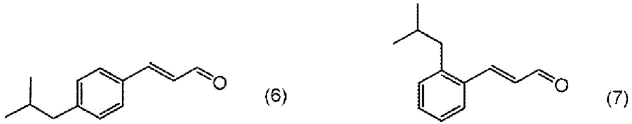

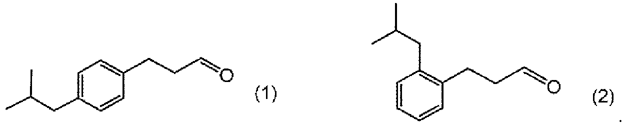

## Patentansprüche

1. Aldehydzusammensetzung, umfassend einen Aldehyd, dargestellt durch die nachstehend beschriebene Formel (1), und einen Aldehyd, dargestellt durch die nachstehend beschriebene Formel (2), wobei das Massenverhältnis [(1)/(2)] zwischen dem Aldehyd der Formel (1) und dem Aldehyd der Formel (2) von 97,00/3,00 bis 99,999/0,001 beträgt.

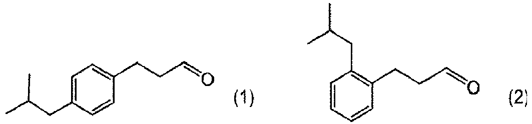

2. Aldehydzusammensetzung nach Anspruch 1, wobei die Zusammensetzung den nachstehend beschriebenen Aldehyd der Formel (1) und de nachstehend beschriebenen Aldehyd der Formel (2) umfasst und das Massenverhältnis [(1)/(2)] zwischen dem Aldehyd der Formel (1) und dem Aldehyd der Formel (2) von 97,00/3,00 bis 99,90/0,10 beträgt.

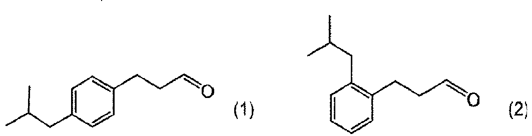

3. Aldehydzusammensetzung nach Anspruch 1, wobei die Zusammensetzung den nachstehend beschriebenen Aldehyd der Formel (1) und den nachstehend beschriebenen Aldehyd der Formel (2) umfasst, das Massenverhältnis [(1)/(2)] zwischen dem Aldehyd der Formel (1) und dem Aldehyd der Formel (2) von 97,00/3,00 bis 99,999/0,001 beträgt und der Gehalt eines nachstehend beschriebenen Esters der Formel (4) 0,10 Masse-% oder weniger, bezogen auf die Gesamtmenge des Aldehyds der Formel (1) und des Aldehyds der Formel (2), beträgt.

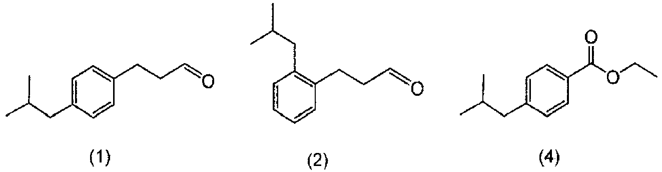

4. Aldehydzusammensetzung nach Anspruch 1, wobei die Zusammensetzung den nachstehend beschriebenen Aldehyd der Formel (1), den nachstehend beschriebenen Aldehyd der Formel (2) und einen nachstehend beschriebenen Ester der Formel (4) umfasst, wobei das Massenverhältnis [(1)/(2)] zwischen dem Aldehyd der Formel (1) und dem Aldehyd der Formel (2) zwischen 97,00/3,00 und 99,999/0,001 liegt und der Gehalt des Esters der Formel (4) 0,50 bis 2,00 Masse-% oder weniger, bezogen auf die Gesamtmenge des Aldehyds der Formel (1) und des Aldehyds der Formel (2), beträgt.

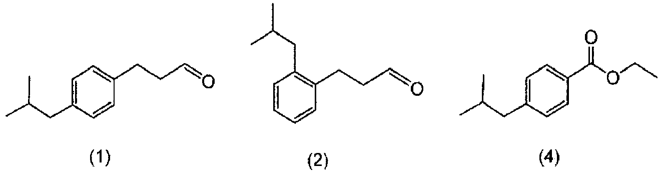

5. Aldehydzusammensetzung nach Anspruch 1, wobei die Gesamtreinheit der durch Formel (1) und Formel (2) dargestellten Aldehyde mindestens 97 Masse- % beträgt.

6. Aldehydzusammensetzung nach Anspruch 1, wobei die Zusammensetzung einen Alkohol der nachstehend beschriebenen Formel (3) in einer Menge von 0,01 bis 0,90 Masse-% enthält.

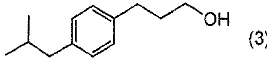

7. Aldehydzusammensetzung nach Anspruch 1, wobei die Zusammensetzung einen Sulfonsäureester der nachstehend beschriebenen Formel (5) in einer Menge von 0,01 bis 0,90 Masse-% enthält.

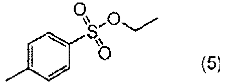

8. Duftstoffzusammensetzung, umfassend die in einem der Ansprüche 1 bis 7 beschriebene Aldehydzusammensetzung.

9. Verfahren zur Herstellung der in Anspruch 1 beschriebenen Aldehydzusammensetzung, wobei das Verfahren in dieser Reihenfolge umfasst:
einen Schritt der Formylierung von Isobutylbenzol mit Kohlenmonoxid unter extrem sauren Bedingungen zur Gewinnung von 4-Isobutylbenzaldehyd und 2- Isobutylbenzaldehyd;
einen Schritt der Aldolkondensation von 4-Isobutylbenzaldehyd und 2-Isobutylbenzaldehyd mit Acetaldehyd zur Gewinnung eines Aldehyds der nachstehend beschriebenen Formel (6) und eines Aldehyds der nachstehend beschriebenen Formel (7); und
einen Schritt der Hydrierung zur Gewinnung eines Aldehyds der nachstehend beschriebenen Formel (1) und eines Aldehyds der nachstehend beschriebenen Formel (2),

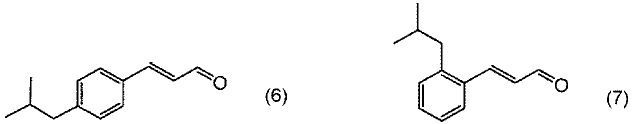

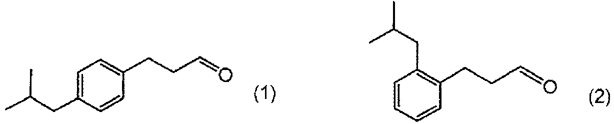

10. Verfahren zur Herstellung der in Anspruch 1 beschriebenen Aldehydzusammensetzung, wobei das Verfahren in dieser Reihenfolge umfasst:
einen Schritt der Formylierung von Isobutylbenzol mit Kohlenmonoxid unter extrem sauren Bedingungen zur Gewinnung von 4-Isobutylbenzaldehyd und 2- Isobutylbenzaldehyd;
einen Schritt der Acetalisierung von 4-Isobutylbenzaldehyd und 2-Isobutylbenzaldehyd in Gegenwart eines para-Toluolsulfonsäure-Katalysators;
einen Schritt, bei dem erhaltene Acetale unter Verwendung eines Säurekatalysators mit einem Alkylvinylether umgesetzt werden;
einen Hydrolyseschritt in Gegenwart eines Säurekatalysators zur Gewinnung eines Aldehyds der nachstehend beschriebenen Formel (6) und eines Aldehyds der nachstehend beschriebenen Formel (7); und
einen Schritt der Hydrierung zur Gewinnung eines Aldehyds der nachstehend beschriebenen Formel (1) und eines Aldehyds der nachstehend beschriebenen Formel (2),

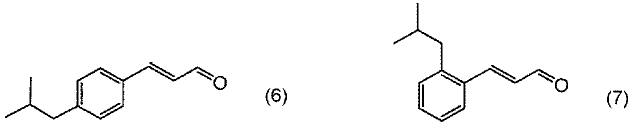

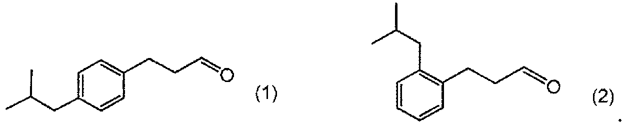

## Revendications

1. Composition d'aldéhyde, comprenant un aldéhyde représenté par la Formule (1) décrite ci-dessous et un aldéhyde représenté par la Formule (2) décrite ci-dessous, dans laquelle un rapport massique [(1)/(2)] entre l'aldéhyde représenté par la Formule (1) et l'aldéhyde représenté par la Formule (2) va de 97,00/3,00 à 99,999/0,001

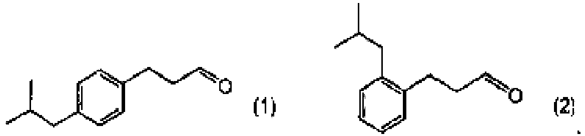

2. Composition d'aldéhyde selon la revendication 1, dans laquelle la composition comprend l'aldéhyde représenté par la Formule (1) décrite ci-dessous et l'aldéhyde représenté par la Formule (2) décrite ci-dessous, et le rapport massique [(1)/(2)] entre l'aldéhyde représenté par la Formule (1) et l'aldéhyde représenté par la Formule (2) va de 97,00/3,00 à 99,90/0,10

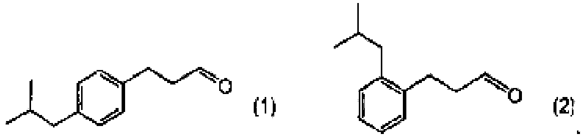

3. Composition d'aldéhyde selon la revendication 1, dans laquelle la composition comprend l'aldéhyde représenté par la Formule (1) décrite ci-dessous et l'aldéhyde représenté par la Formule (2) décrite ci-dessous, le rapport massique [(1)/(2)] entre l'aldéhyde représenté par la Formule (1) et l'aldéhyde représenté par la Formule (2) va de 97,00/3,00 à 99,999/0,001, et une teneur en un ester représenté par la Formule (4) décrite ci-dessous est de 0,10 % en masse ou moins par rapport à une quantité totale de l'aldéhyde représenté par la Formule (1) et l'aldéhyde représenté par la Formule (2)

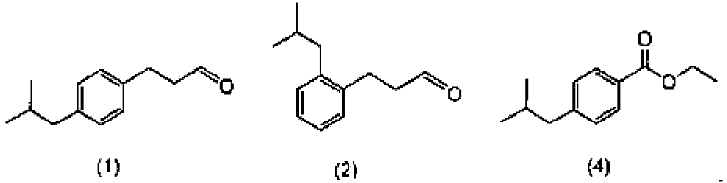

4. Composition d'aldéhyde selon la revendication 1, dans laquelle la composition comprend l'aldéhyde représenté par la Formule (1) décrite ci-dessous, l'aldéhyde représenté par la Formule (2) décrite ci-dessous, un ester représenté par la Formule (4) décrite ci-dessous, le rapport massique [(1)/(2)] entre l'aldéhyde représenté par la Formule (1) et l'aldéhyde représenté par la Formule (2) va de 97,00/3,00 à 99,999/0,001, et une teneur en l'ester représenté par la Formule (4) va de 0,50 à 2,00 % en masse ou moins par rapport à une quantité totale de l'aldéhyde représenté par la Formule (1) et l'aldéhyde représenté par la Formule (2)

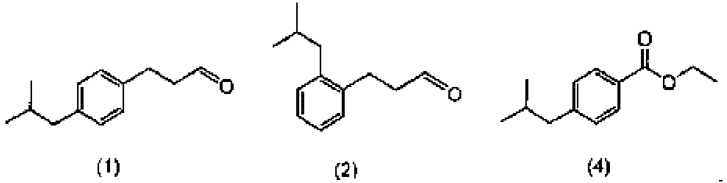

5. Composition d'aldéhyde selon la revendication 1, dans laquelle une pureté totale des aldéhydes représentés par la Formule (1) et la Formule (2) est de 97 % en masse ou plus.

6. Composition d'aldéhyde selon la revendication 1, dans laquelle la composition comprend un alcool représenté par la Formule (3) décrite ci-dessous en une quantité allant de 0,01 à 0,90 % en masse

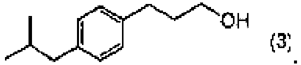

7. Composition d'aldéhyde selon la revendication 1, dans laquelle la composition comprend un ester d'acide sulfonique représenté par la Formule (5) décrite ci-dessous en une quantité allant de 0,01 à 0,90 % en masse

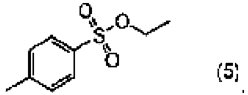

8. Composition de fragrance comprenant la composition d'aldéhyde décrite dans l'une quelconque des revendications 1 à 7.

9. Procédé de production de la composition d'aldéhyde décrite dans la revendication 1, le procédé comprenant, dans cet ordre :
une étape de formylation d'isobutylbenzène avec du monoxyde de carbone dans une condition d'acide super fort pour obtenir du 4-isobutylbenzaldéhyde et 2-isobutylbenzaldéhyde ;
une étape de réalisation d'une condensation aldolique de 4-isobutylbenzaldéhyde et 2-isobutylbenzaldéhyde avec un acétaldéhyde pour obtenir un aldéhyde représenté par la Formule (6) décrite ci-dessous et un aldéhyde représenté par la Formule (7) décrite ci-dessous ; et
une étape de réalisation d'une hydrogénation pour obtenir un aldéhyde représenté par la Formule (1) décrite ci-dessous et un aldéhyde représenté par la Formule (2) décrite ci-dessous

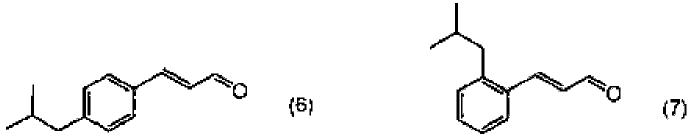

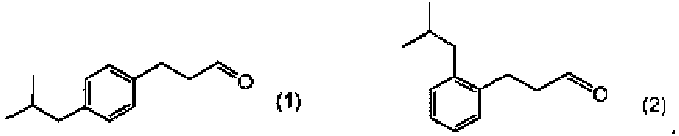

10. Procédé de production de la composition d'aldéhyde décrite dans la revendication 1, le procédé comprenant, dans cet ordre :
une étape de formylation d'isobutylbenzène avec du monoxyde de carbone dans une condition d'acide super fort pour obtenir du 4-isobutylbenzaldéhyde et 2-isobutylbenzaldéhyde ;
une étape d'acétalisation de 4-isobutylbenzaldéhyde et 2-isobutylbenzaldéhyde en présence d'un catalyseur d'acide para-toluènesulfonique ;
une étape d'amenée des acétals obtenus à réagir avec un éther alkylvinylique sous un catalyseur acide ;
une étape d'hydrolyse en présence d'un catalyseur acide pour obtenir un aldéhyde représenté par la Formule (6) décrite ci-dessous et un aldéhyde représenté par la Formule (7) décrite ci-dessous ; et
une étape de réalisation d'une hydrogénation pour obtenir un aldéhyde représenté par la Formule (1) décrite ci-dessous et un aldéhyde représenté par la Formule (2) décrite ci-dessous
